# EUROPEAN PATENT APPLICATION

(11) **EP 4 397 283 A1**
(43) Date of publication of application: **10.07.2024**
(21) Application number: 22864487.8
(22) Date of filing: 29.08.2022
(51) Int. Cl.: A61F 2/88

(54) **STENT AND STENT DELIVERY SYSTEM**

(30) Priority: 31.08.2021 WO PCT/JP2021/032009
(71) Applicant: Olympus Corporation, Hachioji-shi Tokyo 192-8507 (JP)
(72) Inventor: KAN, Kazuhiro, Hachioji-shi, Tokyo 192-8507 (JP); NOGUCHI, Shun, Hachioji-shi, Tokyo 192-8507 (JP); SASAKI, Kanta, Hachioji-shi, Tokyo 192-8507 (JP); TAKITA, Ko, Hachioji-shi, Tokyo 192-8507 (JP); IMAOKA, Yuka, Hachioji-shi, Tokyo 192-8507 (JP)
(74) Representative: Schicker, Silvia
(86) International application number: PCT/JP2022/032380
(87) International publication number: WO 2023/032905

(57) **Abstract**

A stent formed by weaving wires includes: a plurality of straight-line crossing portions, which are formed by crossing at least two straight-line portions of the wires and are arranged adjacent to each other in a circumferential direction of the stent; and a plurality of interlocking portions configured by intersecting a peak-shaped bent portion, in which the wire is bent in a first direction side which is one side of a longitudinal axis direction of the stent and becomes convex, and a valley-shaped bent portion, in which the wire is bent in a second direction side which is the other side of the longitudinal axis direction and becomes convex, and arranged so as to be adjacent to each other in the circumferential direction of the stent, wherein the interlocking portions and the straight-line crossing portions are arranged alternately in the longitudinal axis direction.

## Description

### TECHNICAL FILED

The present invention relates to a stent and a stent delivery system. This application claims priority based on PCT/JP2021/032009 filed on August 31, 2021, the contents of which are hereby incorporated by reference.

### BACKGROUND ART

A procedure is known in which a stent is placed and expanded for stenosis or obstruction (hereinafter referred to as "stenosis, etc.") that occurs in the digestive tract. A stent delivery system is used to place a stent in a stenosis or the like. A stent delivery system passes through a treatment instrument channel of an endoscope to deliver a stent to a stenosis or the like.

For example, the stent described in Patent Document 1 is constructed by winding a single wire around a pin attached to a jig and weaving it like a fence. The stent is formed with a portion (interlocking portion 60) where two curved shapes are interlocked with each other. Therefore, it has features such that it has a high shapefollowability even when it is bent, and easily adapts to the topography of the lumen in which it is placed.

### (Prior Art Document)

### (Patent Document)

(Patent Document 1)
United States Patent No. 6,974,472

### SUMMARY OF INVENTION

### (Problems to be solved by the Invention)

The stent described in Patent Document 1 is constructed by weaving a single wire. Therefore, the portions where the straight wires intersect each other (straight-line crossing portions 70) are positioned adjacent to the interlocking portions 60 in the circumferential direction of the stent. When the stent is bent, the interlocking portion 60 can bend to follow, but the straight-line crossing portion 70 generates a force (axial force) that opposes bending. Therefore, the shape followability of the stent as a whole is impaired. In particular, it is difficult to place a stent in a place where the shape of the lumen is greatly curved. Alternatively, even if the placement is successful, the lumen tends to conform to the shape of the stent, which may put a strain on the lumen, or may cause problems such as the stent moving from the placement position.

In light of the above circumstances, an object of the present invention is to provide a stent that is easy to bend and maintain a bent state, and a stent delivery system that includes the stent.

### (Means for Solving the Problem)

In order to solve the above problems, the present invention proposes the following means.

A stent according to a first aspect of the present invention is a stent formed by weaving wires, including: a plurality of straight-line crossing portions, which are formed by crossing at least two straight-line portions of the wires and are arranged adjacent to each other in a circumferential direction of the stent; and a plurality of interlocking portions configured by intersecting a peak-shaped bent portion, in which the wire is bent in a first direction side which is one side of a longitudinal axis direction of the stent and becomes convex, and a valley-shaped bent portion, in which the wire is bent in a second direction side which is the other side of the longitudinal axis direction and becomes convex, and arranged so as to be adjacent to each other in the circumferential direction of the stent, wherein the interlocking portions and the straight-line crossing portions are arranged alternately in the longitudinal axis direction.

A delivery system according to a second aspect of the present invention includes: an operation portion; an outer tubular member configured to extend distally from the operation portion; an inner tubular member configured to extend distally from the operation portion and located inside the outer tubular member; and the stent which is accommodated between the outer tubular member and the inner tubular member, wherein the operation portion is configured to place the stent by moving the outer tubular member or the inner tubular member in the longitudinal direction.

### (Effect of the Invention)

The stent of the present invention is easy to bend, and it is easy to maintain a bent state.

### BRIEF DESCRIPTION OF THE DRAWINGS

FIG. 1 is a diagram showing the overall configuration of an endoscope system with a stent according to a first embodiment of the present invention.
FIG. 2 is a diagram showing the overall configuration of the stent.
FIG. 3 is a development diagram in which the stent is deployed in the circumferential direction.
FIG. 4 is a development diagram of the stent containing a partial enlarged diagram.
FIG. 5 is a diagram showing a crossing between the first straight line and other wires.
FIG. 6 is a diagram showing an example of a transformation of a wire crossing portion.
FIG. 7 is a diagram showing a crossing between the first line and other wires in the transformation example.
FIG. 8 is a diagram showing other deformation examples of a wire crossing portion.
FIG. 9 is a diagram showing a crossing between the first line and other wires in the transformation example.
FIG. 10 is a development diagram in which a stent according to a second embodiment of the present invention is deployed in the circumferential direction.
FIG. 11 is a diagram showing an example of the arrangement of the placement of the first straight-line crossing portion and the second straight-line crossing portion.
FIG. 12 is a diagram showing other deformation examples of the placement of the first straight-line crossing portion and the second straight-line crossing portion.
FIG. 13 is a diagram showing other deformation examples of the placement of the first straight-line crossing portion and the second straight-line crossing portion.
FIG. 14 is a development diagram in which a stent according to a third embodiment of the present invention is deployed in the circumferential direction.
FIG. 15 is a diagram showing an example of the arrangement of the placement of the first straight-line crossing portion and the second straight-line crossing portion.
FIG. 16 is a diagram showing other deformation examples of the placement of the first straight-line crossing portion and the second straight-line crossing portion.
FIG. 17 is a development diagram in which a stent according to a fourth embodiment of the present invention is deployed in the circumferential direction.
FIG. 18 is a diagram showing an example of the arrangement of the placement of the first straight-line crossing portion and the second straight-line crossing portion.
FIG. 19 is a diagram showing other deformation examples of the placement of the first straight-line crossing portion and the second straight-line crossing portion.
FIG. 20 is a development diagram in which a stent according to a fifth embodiment of the present invention is deployed in the circumferential direction.
FIG. 21 is a diagram showing an example of a deformation of the placement of the first straight-line crossing portion and the second straight-line crossing portion.
FIG. 22 is a diagram showing other deformation examples of the placement of the first straight-line crossing portion and the second straight-line crossing portion.
FIG. 23 is a diagram showing other deformation examples of the placement of the first straight-line crossing portion and the second straight-line crossing portion.
FIG. 24 is a diagram showing other deformation examples of the placement of the first straight-line crossing portion and the second straight-line crossing portion.
FIG. 25 is a diagram showing a deformation example of the placement of a plurality of interlocking portions.
FIG. 26 is a development diagram in which a stent according to a sixth embodiment of the present invention is expanded in the circumferential direction.
FIG. 27 is an enlarged view of the area R3 shown in FIG. 26.
FIG. 28 is a development diagram in which a stent according to a seventh embodiment of the present invention is deployed in the circumferential direction.
FIG. 29 is an enlarged view of the area R4 shown in FIG. 28.
FIG. 30 is a development diagram of a deformation example of the stent.
FIG. 31 is a development diagram in which a stent according to an eighth embodiment of the present invention is expanded in the circumferential direction.
FIG. 32 is a development diagram in which a stent according to a ninth embodiment of the present invention is deployed in the circumferential direction.
FIG. 33 is a development diagram of the first wire that is woven in the stent manufacturing method, developed in the circumferential direction, according to a tenth embodiment of the present invention.
FIG. 34 is a development diagram in which the first wires and second wires are developed in the stent manufacturing method.
FIG. 35 is a diagram showing a stent according to an eleventh embodiment of the present invention.
FIG. 36 is a diagram showing a reduced diameter stent.
FIG. 37 is a development diagram in which the braided first wires are deployed in the circumferential direction in the manufacturing method of the stent.
FIG. 38 is a development diagram in which the braided first wires and second wires are deployed in the circumferential direction in the manufacturing method of the stent.
FIG. 39 is a diagram showing a deformation example of the stent.

### EMBODIMENTS FOR CARRYING OUT THE INVENTION

### (First embodiment)

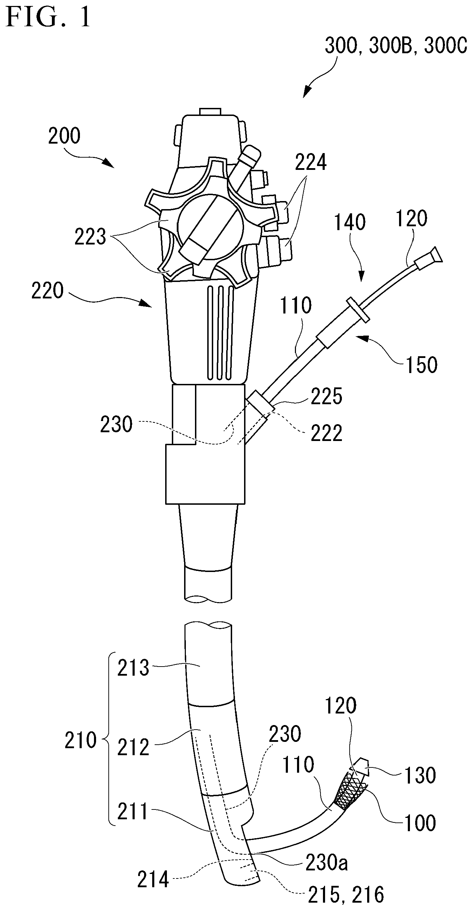

An endoscope system 300 having a stent 100 according to a first embodiment of the present invention will be described with reference to FIGS. 1 to 5. FIG. 1 is a diagram showing the overall configuration of the endoscope system 300.

### [Endoscope system 300]

The endoscope system 300 includes an endoscope 200 and a stent delivery system 150 inserted through a channel of the endoscope 200.

### [Endoscope 200]

The endoscope 200 is a known side-viewing flexible endoscope, and includes an elongated insertion portion 210 and an operation portion 220 provided at the proximal end of the insertion portion 210. Note that the endoscope 200 may be a direct-view flexible endoscope.

The insertion portion 210 includes a distal end rigid portion 211 provided at the distal end portion, a bendable bent portion 212 provided at the proximal end side of the distal end rigid portion 211, and a flexible tube portion 213 provided at the proximal end side of the bent portion 212. An imaging unit 216 having a light guide 215 and a CCD is provided on the side surface of the distal end rigid portion 211 in a state of being exposed to the outside.

The insertion portion 210 is formed with a treatment instrument channel 230 through which an endoscopic treatment instrument such as the stent delivery system 150 is inserted. A distal end portion 230a of the treatment instrument channel 230 is open on the side surface of the distal end rigid portion 211. A proximal end portion of the treatment instrument channel 230 extends to the operation portion 220.

A raising base 214 is provided on the distal end hard portion 211 of the treatment instrument channel 230. A proximal end portion of the raising base 214 is rotatably supported by the distal end rigid portion 211. An elevator operating wire (not shown) fixed to the distal end of the elevator 214 extends through the insertion portion 210 toward the proximal end.

The bent portion 212 is configured to be freely bendable in the vertical and horizontal directions. The distal end of the operation wire is fixed to the distal end side of the bent portion 212. The operation wire extends through the insertion portion 210 to the operation portion 220.

A knob 223 for operating the operation wire and a switch 224 for operating the imaging unit 216 and the like are provided on the proximal end side of the operation portion 220. The user can bend the bent portion 212 in a desired direction by operating the knob 223.

A forceps port 222 that communicates with the treatment instrument channel 230 is provided on the distal end side of the operation portion 220. A user can insert an endoscopic instrument such as the stent delivery system 150 through the forceps port 222. A forceps plug 225 is attached to the forceps port 222 to prevent leakage of bodily fluids.

### [Stent delivery system 150]

The stent delivery system 150 is elongated as a whole and includes the stent 100, an outer tubular member 110, an inner tubular member 120, and an operation portion 140.

The outer tubular member 110 is made of resin or the like in a cylindrical shape and has flexibility. The outer tubular member 110 can be inserted through the treatment instrument channel 230 of the endoscope 200.

The inner tubular member 120 has an outer diameter smaller than the inner diameter of the outer tubular member 110 and can be passed through the inner space (lumen) of the outer tubular member 110. The inner tubular member 120 is made of resin or the like and has flexibility. A tip 130 having an outer diameter larger than that of the outer tubular member 110 is provided at the tip of the inner tubular member 120.

The stent 100 is housed at the distal end of the stent delivery system 150, as shown in FIG. 1. The stent 100 is accommodated in the gap between the inner tubular member 120 and the outer tubular member 110 in a state in which the inner tubular member 120 is passed through the inside thereof and the diameter thereof is reduced.

The operation portion 140 is connected to the proximal end sides of the outer tubular member 110 and the inner tubular member 120, and is configured to allow the outer tubular member 110 to move relative to the inner tubular member 120 in the longitudinal direction. By operating the operation portion, the operator moves the outer tubular member 110 with respect to the inner tubular member 120 to expose the accommodated stent 100, and as a result, the stent 100 can be placed. In addition, when the stent is exposed, the operator can move the outer tubular member 110 in the opposite direction relative to the inner tubular member 120, thereby allowing the stent 100 to be re-accommodated.

### [Stent 100]

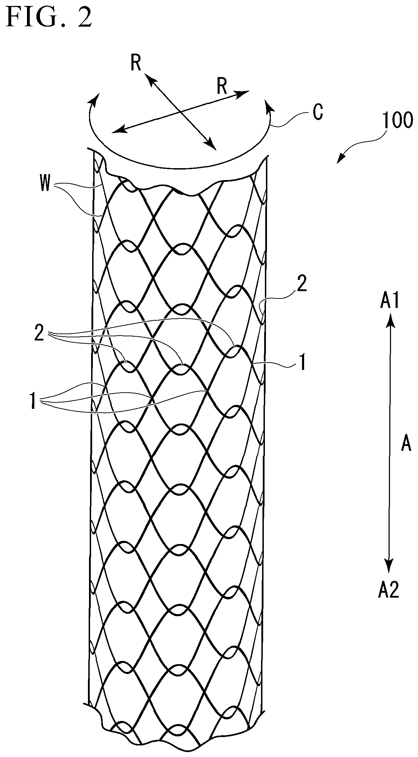

FIG. 2 is a diagram showing the overall configuration of the stent 100.

The stent 100 is formed by weaving wires and has a cylindrical shape. The stent 100 is indwelled in a digestive system body lumen such as the bile duct, esophagus, duodenum, small intestine, large intestine, etc., and is used mainly for the purpose of expanding and maintaining the lumen.

The stent 100 of this embodiment is not a so-called covered stent whose outer peripheral surface side is covered with a resin film or the like, but an uncovered stent that is not covered with a film or the like. However, the stent 100 can also be used as a covered stent by being covered with a resin film or the like.

In the following description, one of the longitudinal axis directions (axial directions) A of the stent 100 is called "first direction A1 ", and the other of the longitudinal axis directions A of the stent 100 is called "second direction A2".

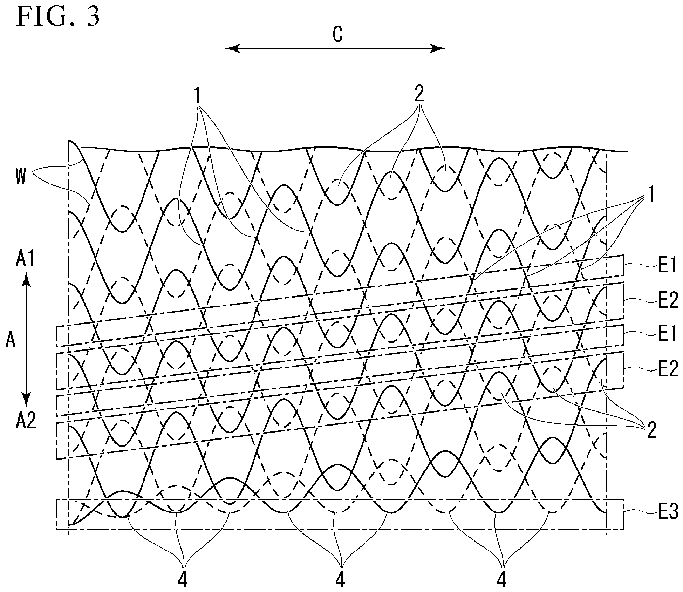

FIG. 3 is a developed view of the stent 100 deployed in the circumferential direction C.

The stent 100 is formed in the shape of a circular tube having meshes on its peripheral surface formed by wires W that extend obliquely in the circumferential direction C while repeatedly bending. The stent 100 has a plurality of straight-line crossing portions 1 and a plurality of interlocking portions 2.

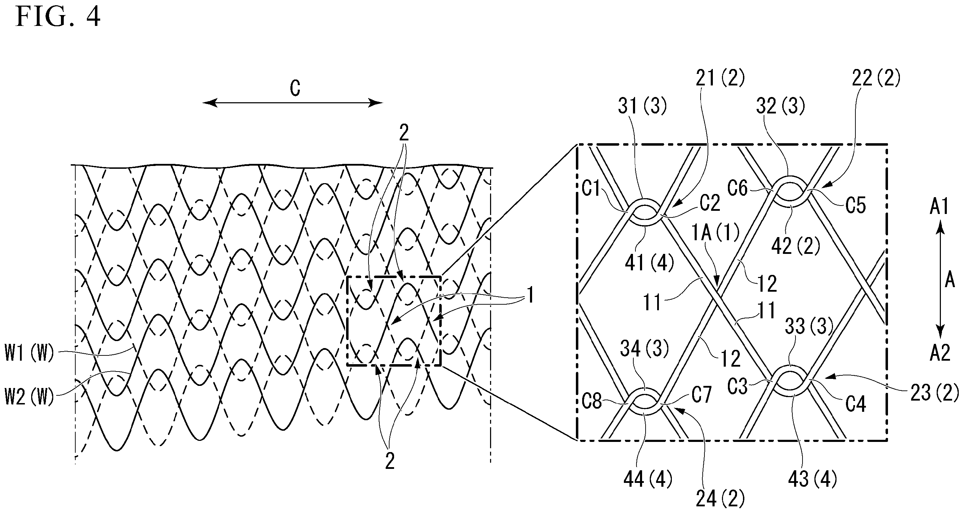

FIG. 4 is an exploded view of the stent 100 including a partially enlarged view.

The straight-line crossing portion 1 is formed by straight-line crossing of straight-line portions 10 of the wire W. Each straight-line portion 10 is a substantially straight-line portion of the wire W and includes a gently curved portion.

Each interlocking portion (engaging portion) 2 is formed by intersecting a peak-shaped bent portion 3 and a valley-shaped bent portion 4. The peak-shaped bent portion (peak) 3 is a convex portion in which the wire W extending obliquely in the circumferential direction C is folded back in the longitudinal axis direction A and convexes in the first direction A1. The valley-shaped bent portion (valley) 4 is a convex portion (concave in the first direction A1 side) in which the wire W extending in the circumferential direction is folded back in the longitudinal direction A and bent in the second direction A2 side (concave in the first direction A1 side). In the interlocking portion 2, the peak-shaped bent portion 3 and the valley-shaped bent portion 4 intersect each other in a hook shape, so that the peak-shaped bent portion 3 and the valley-shaped bent portion 4 are connected so as to be relatively movable although they cannot be separated.

As shown in FIG. 3, first regions E1 in which the plurality of straight-line crossing portions 1 are arranged and second regions E2 in which the plurality of interlocking portions 2 are arranged are alternately arranged in the longitudinal axis direction A. Each first region E1 is spirally arranged along the longitudinal axis direction A. In addition, each second region E2 is spirally arranged along the longitudinal axis direction A.

An end region E3 of the stent 100 on the second direction A2 side is arranged along the circumferential direction C without the valley-shaped bent portion 4 intersecting the peak-shaped bent portion 3. In addition, the end region (not shown) of the stent 100 on the first direction A1 side is arranged along the circumferential direction C without the peak-shaped bent portions 3 intersecting the valley-shaped bent portions 4.

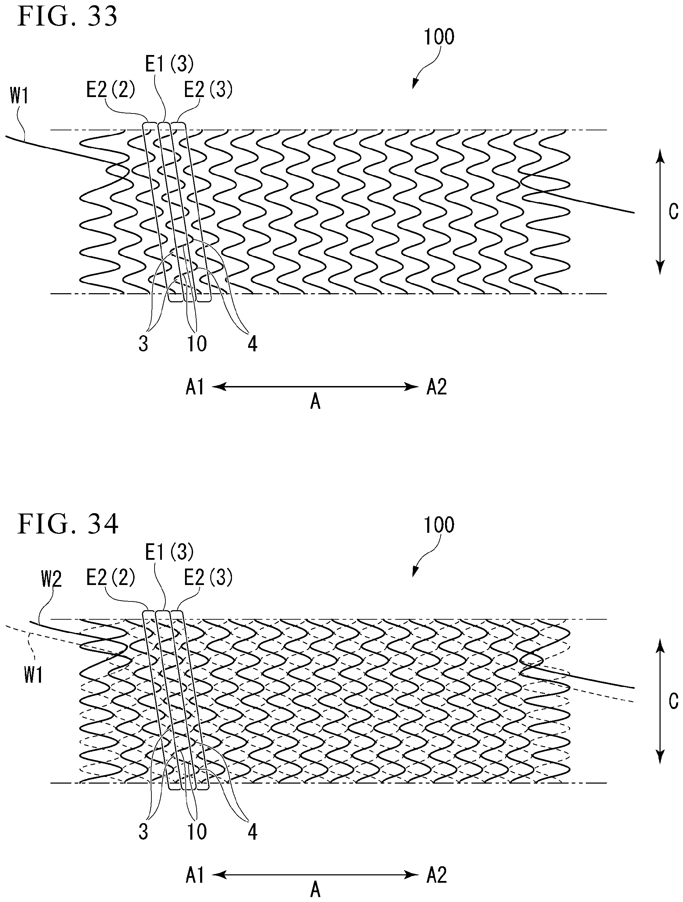

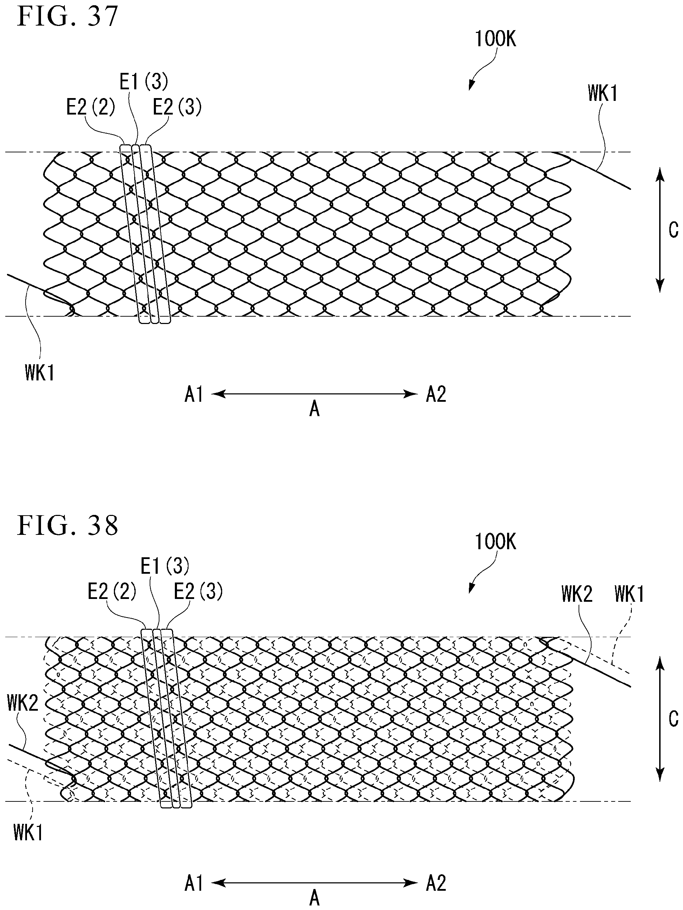

The end portion of the valley-shaped bent portion 4 in the end region E3 on the second direction A2 side may be arranged spirally without intersecting the peak-shaped bent portion 3 as shown in FIG. 3, and may be aligned with respect to the longitudinal axis direction A. The same applies to the ends of the peak-shaped bent portions 3 on the first direction A1 side in the end regions (not shown) of the stent 100 on the first direction A1 side. For example, as in the end regions of the stent 100 shown in FIG. 34 and the end region of a stent 100K shown in FIG. 38, the positions of the ends with respect to the longitudinal axis direction A can be aligned by adjusting the length of the longitudinal axis direction A of the peak-shaped bent portion 3 and the valley-shaped bent portion 4 in the end region.

### [Central straight-line crossing portion 1A]

A first straight-line portion 11 and a second straight-line portion 12, which are the straight-line portions 10, intersect at the "central straight-line crossing portion 1A", which is the straight-line crossing portion 1, as shown in FIG. 4. Specifically, when viewed from the radial direction R (see FIG. 2) of the stent 100, the first straight-line portion 11 and the second straight-line portion 12 intersect at the central straight-line crossing portion 1A. At the central straight-line crossing portion 1A, the first straight-line portion 11 passes outside the second straight-line portion 12 in the radial direction R.

### [First interlocking portion 21]

On the first direction A1 side of the first straight-line portion 11, a "first peak 31", which is the peak-shaped bent portion 3, continues. The first peak 31 intersects with a first valley 41 that is the valley-shaped bent portion 4 to form the "first interlocking portion 2" that is the interlocking portion 2.

Specifically, when viewed from the radial direction R of the stent 100, the first peak 31 and the first valley 41 intersect at a first crossing portion C1 and a second crossing portion C2 closer to the central straight-line crossing portion 1A than the first crossing portion C1. At the first crossing portion C1, the first peak 31 passes through the outer side of the first valley 41 in the radial direction R. At the second crossing portion C2, the first peak 31 passes through the inner side of the first valley 41 in the radial direction R.

### [Second interlocking portion 22]

A "second peak 32", which is the peak-shaped bent portion 3, is connected to the first direction A1 side of the second straight-line portion 12. The second peak 32 intersects with a second valley 42 that is the valley-shaped bent portion 4 to form the "second interlocking portion 22" that is the interlocking portion 2.

Specifically, when viewed from the radial direction R of the stent 100, the second peak 32 and the second valley 42 intersect at a fifth crossing portion C5 and a sixth crossing portion C6 closer to the central straight-line crossing portion 1A than the fifth crossing portion C5. At the fifth crossing portion C5, the second peak 32 passes inside the second valley 42 in the radial direction R. At the sixth crossing portion C6, the second peak 32 passes outside the second valley 42 in the radial direction R.

### [Third interlocking portion 23]

On the second direction A2 side of the first straight-line portion 11, a "third valley 43", which is the valley-shaped bent portion 4, continues. The third valley 43 intersects with a third peak 33 that is the peak-shaped bent portion 3 to form the "third interlocking portion 23" that is the interlocking portion 2.

Specifically, when viewed from the radial direction R of the stent 100, the third peak 33 and the third valley 43 intersect at a fourth crossing portion C4 and a third crossing portion C3 closer to the central straight-line crossing portion 1A than the fourth crossing portion C4. The third valley 43 passes inside the third peak 33 in the radial direction R at the third crossing portion C3. The third valley 43 passes outside the third peak 33 in the radial direction R at the fourth crossing portion C4.

### [Fourth interlocking portion 24]

On the second direction A2 side of the second straight-line portion 12, a "fourth valley 44", which is the valley-shaped bent portion 4, continues. The fourth valley 44 intersects with a fourth peak 34 that is the peak-shaped bent portion 3 to form the "fourth interlocking portion 24" that is the interlocking portion 2.

Specifically, when viewed from the radial direction R of the stent 100, the fourth peak 34 and the fourth valley 44 intersect at an eighth crossing portion C8 and a seventh crossing portion C7 closer to the central straight-line crossing portion 1A than the eighth crossing portion C8. The fourth valley 44 passes outside the fourth peak 34 in the radial direction R at the seventh crossing portion C7. The fourth valley 44 passes inside the fourth peak 34 in the radial direction R at the fourth crossing portion C4.

### [Arrangement of straight-line crossing portion 1 and interlocking portion 2]

The first interlocking portion 21 and the second interlocking portion 22 are arranged at different positions in the longitudinal axis direction A. Specifically, the second interlocking portion 22 is arranged on the first direction A1 side of the first interlocking portion 21 in the longitudinal axis direction A. Also, the first interlocking portion 21 is arranged in the longitudinal axis direction A between the second interlocking portion 22 and the central straight-line crossing portion 1A.

The third interlocking portion 23 and the fourth interlocking portion 24 are arranged at different positions in the longitudinal axis direction A. Specifically, the third interlocking portion 23 is arranged on the first direction A1 side of the fourth interlocking portion 24 in the longitudinal axis direction A. Further, the third interlocking portion 23 is arranged between the central straight-line crossing portion 1A and the fourth interlocking portion 24 in the longitudinal axis direction A.

The central straight-line crossing portion 1A is arranged between the first interlocking portion 21 and the second interlocking portion 22 in the circumferential direction C. Further, the central straight-line crossing portion 1A is arranged between the third interlocking portion 23 and the fourth interlocking portion 24 in the circumferential direction C.

### [Wire W]

The first peak 31, the first straight-line portion 11, and the third valley 43 are continuous parts of the wire W extending in a zigzag along the circumferential direction C, and are parts of the wire W indicated by broken lines in FIG. 4. The wire W indicated by the dashed line in FIG. 4 is also referred to as "first wire W1".

The second peak 32, the second straight-line portion 12, and the fourth valley 44 are continuous parts of the wire W extending in a zigzag along the circumferential direction C, and are parts of the wire W indicated by solid lines in FIG. 4. The wire W indicated by the solid line in FIG. 4 is also referred to as "second wire W2".

The first wire W1 and the second wire W2 may be one continuous wire, or may be different wires.

### [Crossing portion of wires W]

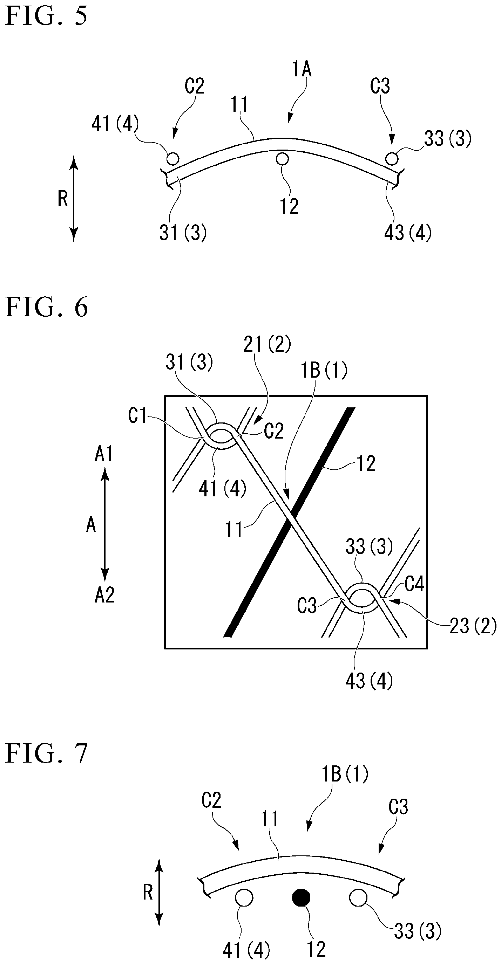

FIG. 5 is a diagram showing the crossing portion of the first straight-line portion 11 and another wire W.

In the first straight-line portion 11, the first peak 31 continuous in the first direction A1 passes inside the first valley 41 in the radial direction R at the second crossing portion C2. Further, in the first straight-line portion 11, the third valley 43 continuous in the second direction A2 passes inside the third peak 33 in the radial direction R at the third crossing portion C3. Therefore, as shown in FIG. 5, the first straight-line portion 11 sandwiched between the second crossing portion C2 and the third crossing portion C3 has a convex shape. As a result, the frictional force of the wires W intersecting at the second crossing portion C2, the third crossing portion C3 and the central straight-line crossing portion 1A increases, and the stent 100 tends to maintain its bent state.

### [Other straight-line crossing portions 1 and interlocking portions 2]

The central straight-line crossing portion 1A and the four interlocking portions 2 (the first interlocking portion 21, the second interlocking portion 22, the third interlocking portion 23 and the fourth interlocking portion 24) connected to the central straight-line crossing portion 1A have the above configuration. The other straight-line crossing portion 1 in the stent 100 and the four interlocking portions 2 connected to the straight-line crossing portion 1 have the same configuration.

### [Operation of stent delivery system 150]

A method for placing a stent using the endoscope system 300 including the stent delivery system 150 will be described by taking a procedure for placing the stent 100 in the bile duct as an example.

The operator inserts the insertion portion 210 of the endoscope 200 into the patient's body cavity through a natural opening such as the mouth. At that time, the operator bends the bent portion 212 by operating the knob 223 or the like as necessary.

The operator passes the guidewire through the treatment instrument channel 230 of the endoscope 200 and inserts the guidewire into the bile duct while observing with the endoscope 200. Subsequently, the operator operates the guidewire under X-ray fluoroscopy to break through the narrowed site in the bile duct, and moves the distal end of the guidewire to the liver side of the narrowed site (target position).

The operator inserts the proximal end of the guide wire protruding from the forceps plug 225 of the endoscope 200 into a through-hole of the tip 130 of the stent delivery system 150.

The operator advances the stent delivery system 150 along the guidewire by pushing the stent delivery system 150 while holding the guidewire. The distal end of stent delivery system 150 protrudes from the distal end of treatment instrument channel 230 of endoscope 200. After the distal end of the stent delivery system 150 breaks through the stenosis site (target position), the operator advances and retracts the stent delivery system 150 to determine the indwelling position of the stent 100. Note that the operator may insert the stent delivery system 150 into the treatment instrument channel 230 without using a guide wire.

After determining the target position of the stent 100, the operator retracts the outer tubular member 110 with respect to the inner tubular member 120. As a result, as shown in FIG. 1, the stent 100 is gradually exposed and expanded from the distal end side.

When the stent 100 is completely exposed, the stent 100 expands as a whole and the inner diameter of the stent 100 becomes larger than the outer diameter of the inner tubular member 120. Along with this, the locking between the stent 100 and the inner tubular member 120 is released.

After the locking between the stent 100 and the inner tubular member 120 is released, when the operator retracts the inner tubular member 120, the stent 100 remains at the indwelling position and the inner tubular member 120 is removed from the stent 100.

When the operator pulls out the stent delivery system 150 excluding the stent 100 from the body, the placement procedure of the stent 100 is completed.

The stent 100 of the present embodiment has a plurality of interlocking portions 2 and has high shape followability even when bent. Further, as shown in FIG. 5, since the first straight-line portion 11 sandwiched between the second crossing portion C2 and the third crossing portion C3 is convex, the frictional force of the wires W intersecting at the second crossing portion C2, the third crossing portion C3, and the central straight-line crossing portion 1A is increased, and the stent 100 can easily maintain its bent state. As a result, the stent 100 is easy to bend and easy to maintain in a bent state.

According to the stent 100 of the present embodiment, the second regions E2 are arranged in a spiral shape, so the stent 100 can be knitted without arranging the straight-line crossing portions 1 in the second regions E2. That is, in the stent 100, it is not necessary to arrange the straight-line crossing portion 1 at a position adjacent to the interlocking portion 2 in the circumferential direction C. Therefore, in the second region E2, the stent 100 has only the interlocking portions 2 having high conformability, and is easy to bend. The first regions E1 where the straight-line crossing portions 1 have a large frictional force (locking force) between the intersecting wires W are arranged alternately with the second regions E2 in the longitudinal axis direction A. Therefore, the stent 100 can preferably maintain its curved shape.

The first embodiment of the present invention has been described above in detail with reference to the drawings, but the specific configuration is not limited to this embodiment, and design changes and the like are included within the scope of the present invention. Also, the constituent elements shown in the above-described embodiment and modifications shown below can be combined as appropriate.

### (Modification 1-1)

The crossing mode of the wires W is not limited to the crossing mode shown in FIG. 5. FIG. 6 is a diagram showing a modification of the intersecting manner of the wires W. FIG. 7 is a diagram showing crossing portions of the first straight-line portion 11 and another wire W in the modification. In the modification shown in FIGS. 6 and 7, the first straight-line portion 11 has the first peak 31 continuous in the first direction A1 passing outside the first valley 41 in the radial direction R at the second crossing portion C2. Further, in the first straight-line portion 11, the third valley 43 continuous in the second direction A2 passes outside the third peak 33 in the radial direction R at the third crossing portion C3. Therefore, as shown in FIG. 7, the first straight-line portion 11 sandwiched between the second crossing portion C2 and the third crossing portion C3 has an arc shape. As a result, the frictional force of the wires W intersecting at the second crossing portion C2, the third crossing portion C3 and the central straight-line crossing portion 1B becomes smaller than in the above embodiment, and the stent 100 becomes more bendable.

### (Modification 1-2)

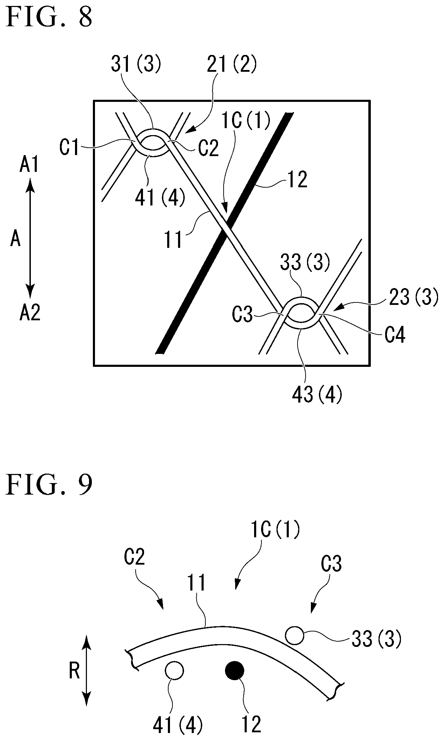

FIG. 8 is a diagram showing another modification of the wire W crossing mode. FIG. 9 is a diagram showing crossing portions of the first straight-line portion 11 and another wire W in another modification. In the modification shown in FIGS. 8 and 9, the first straight-line portion 11 has the first peak 31 continuous in the first direction A1 passing outside the first valley 41 in the radial direction R at the second crossing portion C2. Further, in the first straight-line portion 11, the third valley 43 continuous in the second direction A2 passes inside the third peak 33 in the radial direction R at the third crossing portion C3. As a result, the frictional force of the wires W intersecting at the second crossing portion C2, the third crossing portion C3, and the central straight-line crossing portion 1C becomes smaller than in the above embodiment, and larger than that in Modification 1-1.

### (Second embodiment)

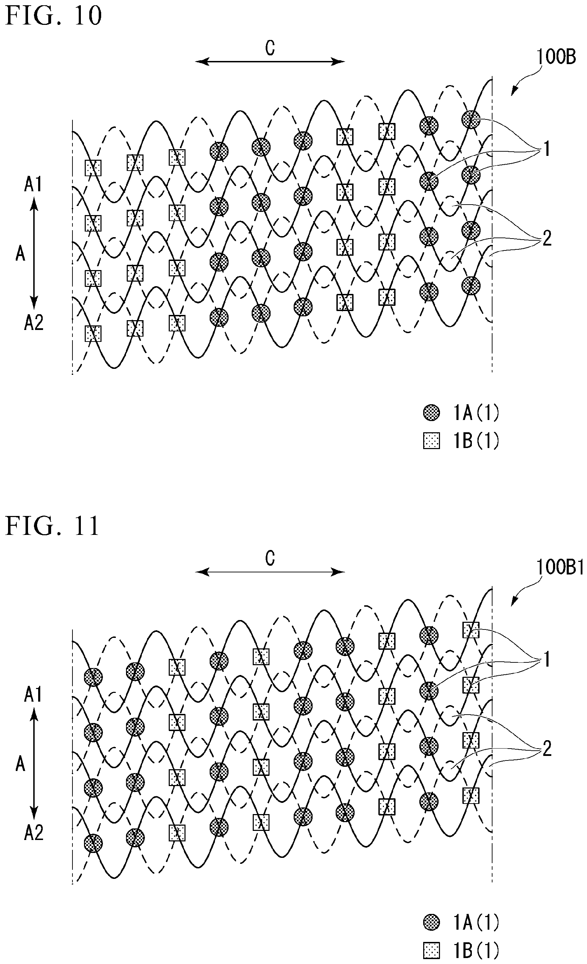

A second embodiment of the present invention will be described with reference to FIG. 10. In the following description, the same reference numerals are given to the same configurations as those already described, and redundant descriptions will be omitted. A stent 100B according to the second embodiment is housed in a stent delivery system 150, like the stent 100 according to the first embodiment.

In the stent 100 according to the first embodiment, the crossing portions of the wires W at the straight-line crossing portion 1 and the four interlocking portions 2 connected to the straight-line crossing portion 1 are the same at any location. In the stent 100B according to the second embodiment, the crossing manner of the wire W at the straight-line crossing portion 1 and the four interlocking portions 2 connected to the straight-line crossing portion 1 differs depending on the location.

### [First straight-line crossing portion 1A]

The central straight-line crossing portion 1A of the stent 100 according to the first embodiment will be referred to as "first straight-line crossing portion 1A" in the following description. Specifically, as shown in FIG. 5, in the first straight-line portion 11 forming the first straight-line crossing portion 1A, the first peak 31 extending in the first direction A1 passes inside the first valley 41 in the radial direction R at the second crossing portion C2. In addition, in the first straight-line portion 11 forming the first straight-line crossing portion 1A, the third valley 43 extending in the second direction A2 passes inside the third peak 33 in the radial direction R at the third crossing portion C3.

### [Second straight-line crossing portion 1B]

The central straight-line crossing portion 1B shown in Modification 1-1 of the first embodiment will be referred to as "second straight-line crossing portion 1B" in the following description. Specifically, as shown in FIG. 7, the first straight-line portion 11 forming the second straight-line crossing portion 1B has a first peak 31 extending in the first direction A1 and a first valley at the second crossing portion C2. 41 in the radial direction R. In addition, in the first straight-line portion 11 forming the second straight-line crossing portion 1B, the third valley 43 continuing on the second direction A2 side passes through the outside of the third peak 33 in the radial direction R at the third crossing portion C3.

The second straight-line crossing portion 1B may be a straight-line crossing portion 1 having a different wire W crossing mode such as the central straight-line crossing portion 1C shown in Modification 1-2.

FIG. 10 is a developed view of the stent 100B deployed in the circumferential direction C.

The stent 100B has a plurality of straight-line crossing portions 1 and a plurality of interlocking portions 2, like the stent 100 according to the first embodiment. The plurality of straight-line crossing portions 1 include a first straight-line crossing portion 1A and a second straight-line crossing portion 1B.

The first straight-line crossing portion 1A is arranged continuously in the longitudinal axis direction A. Two or three first straight-line crossing portions 1A are continuously arranged in the circumferential direction C. Moreover, the second straight-line crossing portion 1B is arranged continuously in the longitudinal axis direction A. Two or three second straight-line crossing portions 1B are continuously arranged in the circumferential direction C. Further, the first straight-line crossing portions 1A and the second straight-line crossing portions 1B are alternately arranged in the circumferential direction C.

In the stent 100B, the number of first straight-line crossing portions 1A and the number of second straight-line crossing portions 1B are substantially equal.

According to the stent 100B of this embodiment, the first straight-line crossing portion 1A where the frictional force of the crossing wires W is high is arranged continuously in the longitudinal axis direction A. Further, the second straight-line crossing portion 1B where the frictional force of the crossing wires W is low is arranged continuously in the longitudinal axis direction A. Further, the first straight-line crossing portions 1A and the second straight-line crossing portions 1B are alternately arranged in the circumferential direction C. As a result, the stent 100B can achieve both high shape followability and high shape retention.

The second embodiment of the present invention has been described above in detail with reference to the drawings, but the specific configuration is not limited to this embodiment, and design changes and the like are also included within the scope of the present invention. Also, the constituent elements shown in the above-described embodiment and modifications can be combined as appropriate.

### (Modification 2-1)

The arrangement of the first straight-line crossing portion 1A and the second straight-line crossing portion 1B is not limited to the arrangement shown in FIG. 10. FIG. 11 is a diagram showing a stent 100B1 that is a modification of the arrangement of the first straight-line crossing portion 1A and the second straight-line crossing portion 1B. In the stent 100B1, the number of the first straight-line crossing portions 1A is greater than the number of the second straight-line crossing portions 1B. Therefore, in the stent 100B 1, the frictional force of the intersecting wires W is higher than in the stent 100B, and the stent 100B 1 is less likely to be crushed in the longitudinal direction A.

### (Modification 2-2)

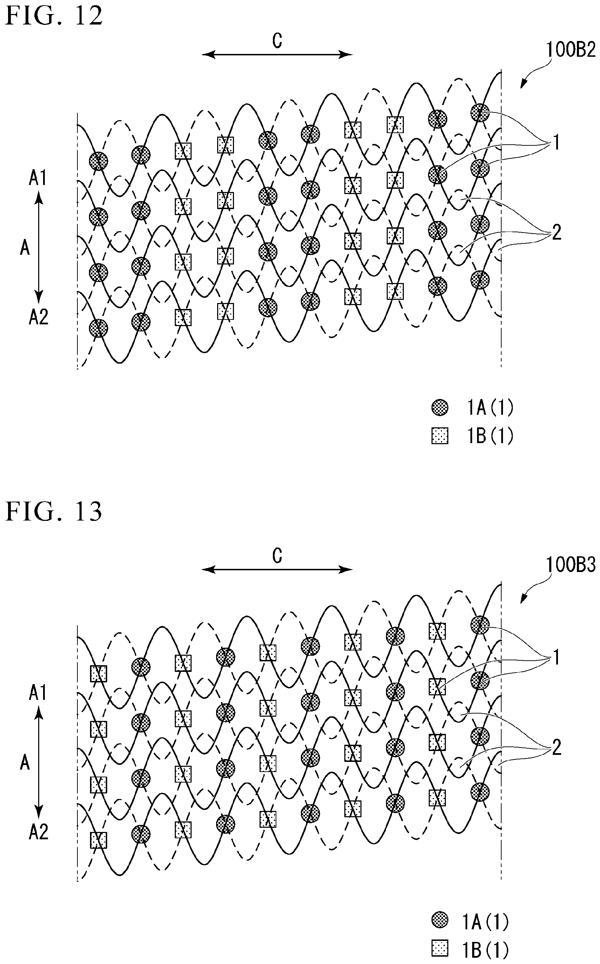

FIG. 12 is a diagram showing a stent 100B2 that is a modification of the arrangement of the first straight-line crossing portion 1A and the second straight-line crossing portion 1B. Two first straight-line crossing portions 1A are continuously arranged in the circumferential direction C. Further, two second straight-line crossing portions 1B are continuously arranged in the circumferential direction C.

### (Modification 2-3)

FIG. 13 is a diagram showing a stent 100B3 that is a modification of the arrangement of the first straight-line crossing portion 1A and the second straight-line crossing portion 1B. The first straight-line crossing portion 1A and the second straight-line crossing portion 1B are arranged alternately one by one in the circumferential direction C. The stent 100B3 has higher shape followability and shape retention than the stent 100B.

### (Third embodiment)

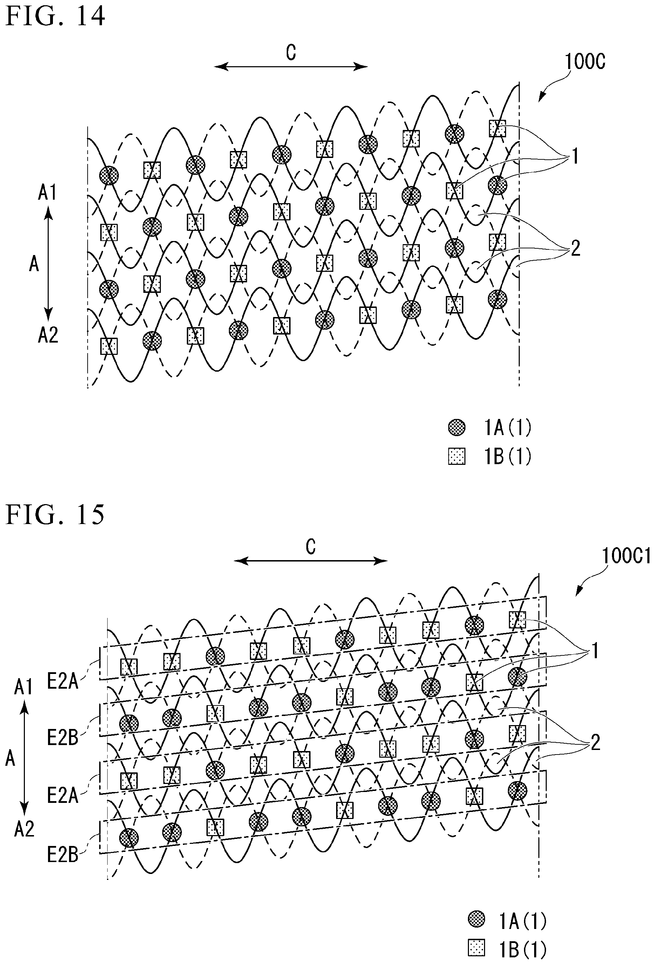

A third embodiment of the present invention will be described with reference to FIG. 14. In the following description, the same reference numerals are given to the same configurations as those already described, and redundant descriptions will be omitted. A stent 100C according to the third embodiment differs from the stent 100B according to the second embodiment only in the arrangement of the first straight-line crossing portion 1A and the second straight-line crossing portion 1B.

FIG. 14 is a developed view of the stent 100C deployed in the circumferential direction C.

The stent 100C has the plurality of straight-line crossing portions 1 and the plurality of interlocking portions 2, like the stent 100 according to the first embodiment. The plurality of straight-line crossing portions 1 include a first straight-line crossing portion 1A and a second straight-line crossing portion 1B.

The first straight-line crossing portion 1A and the second straight-line crossing portion 1B are alternately arranged one by one in the longitudinal axis direction A. Also, the first straight-line crossing portion 1A and the second straight-line crossing portion 1B are alternately arranged in the circumferential direction C one by one.

According to the stent 100C of this embodiment, the first straight-line crossing portion 1A where the frictional force of the crossing wires W is high and the second straight-line crossing portion 1B where the frictional force of the crossing wires W is low are alternately arranged both in the longitudinal direction A and in the circumferential direction C. Therefore, it is possible to achieve both high shape followability and high shape maintainability.

The third embodiment of the present invention has been described above in detail with reference to the drawings, but the specific configuration is not limited to this embodiment, and design changes and the like are included within the scope of the present invention. Also, the constituent elements shown in the above-described embodiment and modifications can be combined as appropriate.

### (Modification 3-1)

The arrangement of the first straight-line crossing portion 1A and the second straight-line crossing portion 1B is not limited to the arrangement shown in FIG. 14. FIG. 15 is a diagram showing a stent 100C1 that is a modification of the arrangement of the first straight-line crossing portion 1A and the second straight-line crossing portion 1B. In the stent 100C1, the first straight-line crossing portion 1A and the second straight-line crossing portion 1B are alternately arranged in the longitudinal axis direction A one by one.

The stent 100C1 includes a second region E2A in which one first straight-line crossing portion 1A and two continuous second straight-line crossing portions 1B are arranged in the circumferential direction C, and a second region E2B in which two consecutive first straight-line crossing portions 1A and one second straight-line crossing portion 1B are arranged in the circumferential direction C. The second region E2A and the second region E2B are alternately arranged in the longitudinal direction A one by one. The stent 100C1 has higher shape followability than the stent 100C.

### (Modification 3-2)

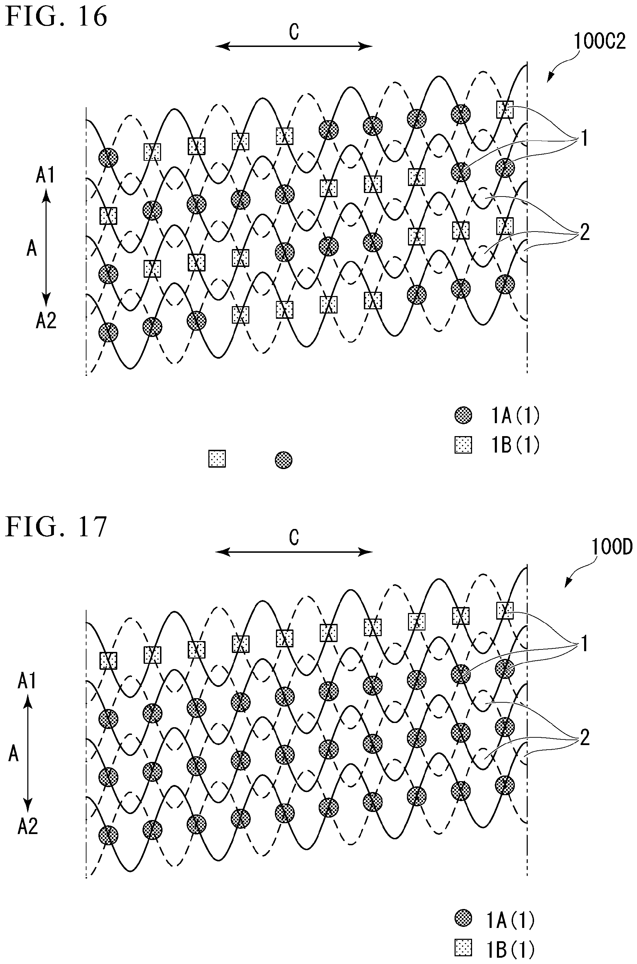

FIG. 16 is a diagram showing a stent 100C2 that is a modification of the arrangement of the first straight-line crossing portion 1A and the second straight-line crossing portion 1B. Three or four first straight-line crossing portions 1A are continuously arranged in the circumferential direction C. In addition, three or four second straight-line crossing portions 1B are continuously arranged in the circumferential direction C. The stent 100C2 can achieve both high shape followability and high shape retention.

### (Fourth embodiment)

A fourth embodiment of the present invention will be described with reference to FIG. 17. In the following description, the same reference numerals are given to the same configurations as those already described, and redundant descriptions will be omitted. A stent 100D according to the fourth embodiment differs from the stent 100B according to the second embodiment only in the arrangement of the first straight-line crossing portion 1A and the second straight-line crossing portion 1B.

FIG. 17 is a developed view of the stent 100D deployed in the circumferential direction C.

The stent 100D has a plurality of straight-line crossing portions 1 and a plurality of interlocking portions 2, like the stent 100 according to the first embodiment. The plurality of straight-line crossing portions 1 include a first straight-line crossing portion 1A and a second straight-line crossing portion 1B.

The first straight-line crossing portions 1A are arranged continuously in the circumferential direction C. Moreover, the second straight-line crossing portion 1B is arranged continuously in the circumferential direction C. The first straight-line crossing portions 1A and the second straight-line crossing portions 1B are alternately arranged in the longitudinal axis direction A.

According to the stent 100D of the present embodiment, the first straight-line crossing portions 1A where the frictional force of the crossing wires W is high are arranged continuously in the circumferential direction C, so that it is easy to maintain a certain shape retention. As a result, the stent 100D can partially retain a portion having a very high shape followability while maintaining a certain shape retention property.

The fourth embodiment of the present invention has been described above in detail with reference to the drawings, but the specific configuration is not limited to this embodiment, and design changes and the like are included within the scope of the present invention. Also, the constituent elements shown in the above-described embodiment and modifications can be combined as appropriate.

### (Modification 4-1)

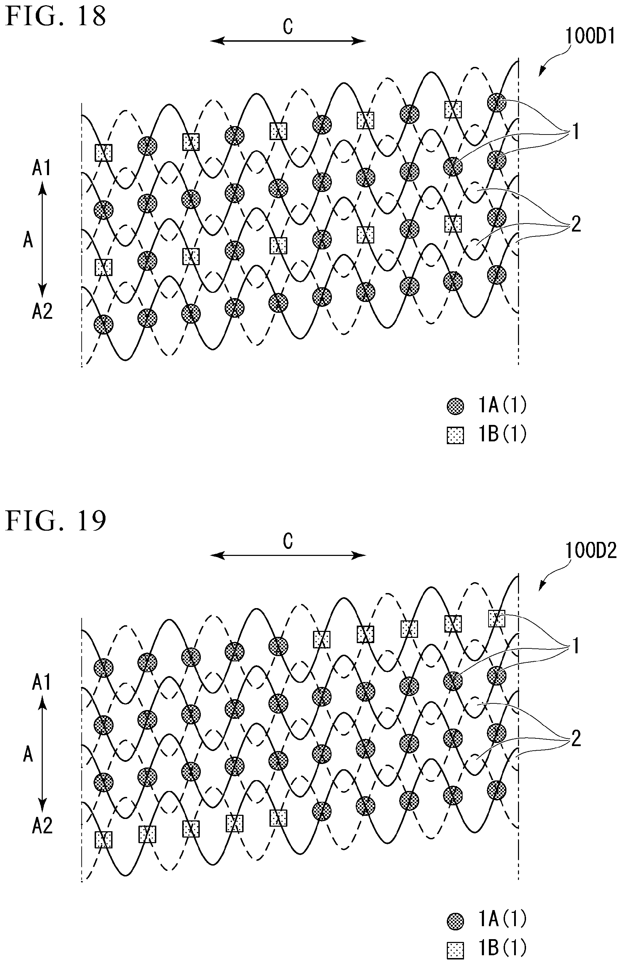

The arrangement of the first straight-line crossing portion 1A and the second straight-line crossing portion 1B is not limited to the arrangement shown in FIG. 17. FIG. 18 is a diagram showing a stent 100D1 that is a modification of the arrangement of the first straight-line crossing portion 1A and the second straight-line crossing portion 1B. In the stent 100D1, the first straight-line crossing portions 1A are alternately arranged in the circumferential direction C. In the stent 100D1, only the second straight-line crossing portion 1B is not arranged continuously in the circumferential direction C. The second straight-line crossing portions 1B and the first straight-line crossing portions 1A are alternately arranged in the circumferential direction C one by one. The stent 100D1 can partially retain a portion with extremely high shape followability while maintaining constant shape retention.

### (Modification 4-2)

FIG. 19 is a diagram showing a stent 100D2 that is a modification of the arrangement of the first straight-line crossing portion 1A and the second straight-line crossing portion 1B. In the stent 100D2, the first straight-line crossing portions 1A are arranged continuously in the circumferential direction C. In the stent 100D2, only the second straight-line crossing portion 1B is not continuously arranged in the circumferential direction C. The five continuous second straight-line crossing portions 1B are arranged adjacent to the five continuous first straight-line crossing portions 1A in the circumferential direction C. In the stent 100D2, the part where the second straight-line crossing portion 1B where the frictional force of the intersecting wires W is low is arranged intensively becomes a part that is easily curved. The stent 100D2 can partially enhance shape followability while maintaining high shape retention in a curved state.

### (Fifth embodiment)

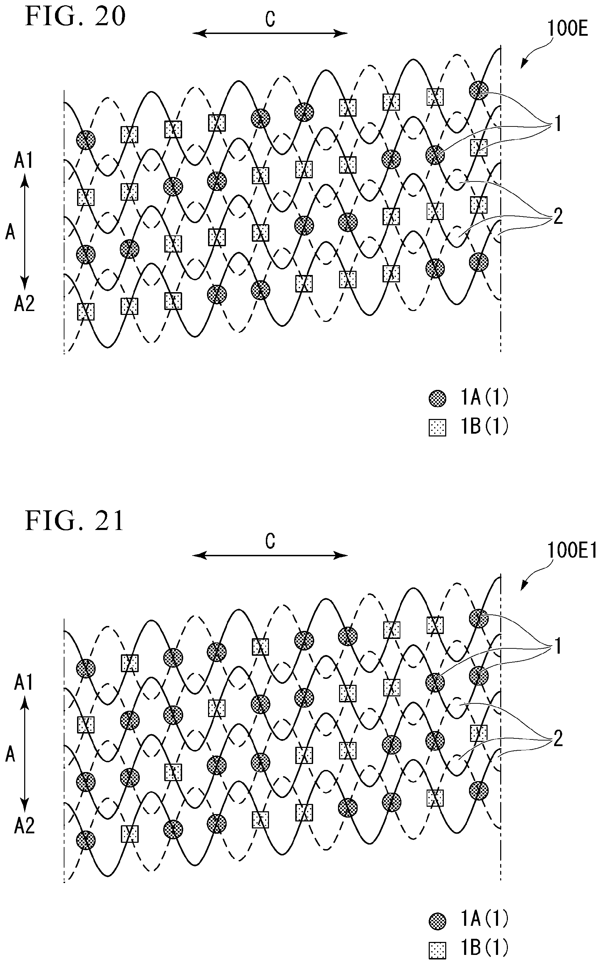

A fifth embodiment of the present invention will be described with reference to FIG. 20. In the following description, the same reference numerals are given to the same configurations as those already described, and redundant descriptions will be omitted. A stent 100E according to the fifth embodiment differs from the stent 100B according to the second embodiment only in the arrangement of the first straight-line crossing portion 1A and the second straight-line crossing portion 1B.

FIG. 20 is a developed view of the stent 100E deployed in the circumferential direction C.

The stent 100E has a plurality of straight-line crossing portions 1 and a plurality of interlocking portions 2, like the stent 100 according to the first embodiment. The plurality of straight-line crossing portions 1 include a first straight-line crossing portion 1A and a second straight-line crossing portion 1B.

The two first straight-line crossing portions 1A continuous in the circumferential direction C are arranged spirally along the longitudinal axis direction A. Further, three second straight-line crossing portions 1B continuous in the circumferential direction C are arranged spirally along the longitudinal axis direction A.

According to the stent 100E of this embodiment, since the first straight-line crossing portion 1A and the second straight-line crossing portion 1B are arranged in a spiral shape along the longitudinal axis direction A, it is possible to prevent twisting and improve shape followability.

The fifth embodiment of the present invention has been described above in detail with reference to the drawings, but the specific configuration is not limited to this embodiment, and design changes and the like are included within the scope of the present invention. Also, the constituent elements shown in the above-described embodiment and modifications can be combined as appropriate.

### (Modification 5-1)

The arrangement of the first straight-line crossing portion 1A and the second straight-line crossing portion 1B is not limited to the arrangement shown in FIG. 20. FIG. 21 is a diagram showing a stent 100E1 that is a modification of the arrangement of the first straight-line crossing portion 1A and the second straight-line crossing portion 1B. Two first straight-line crossing portions 1A continuous in the circumferential direction C are arranged spirally along the longitudinal axis direction A. Two second straight-line crossing portions 1B continuous in the circumferential direction C are arranged spirally along the longitudinal axis direction A.

### (Modification 5-2)

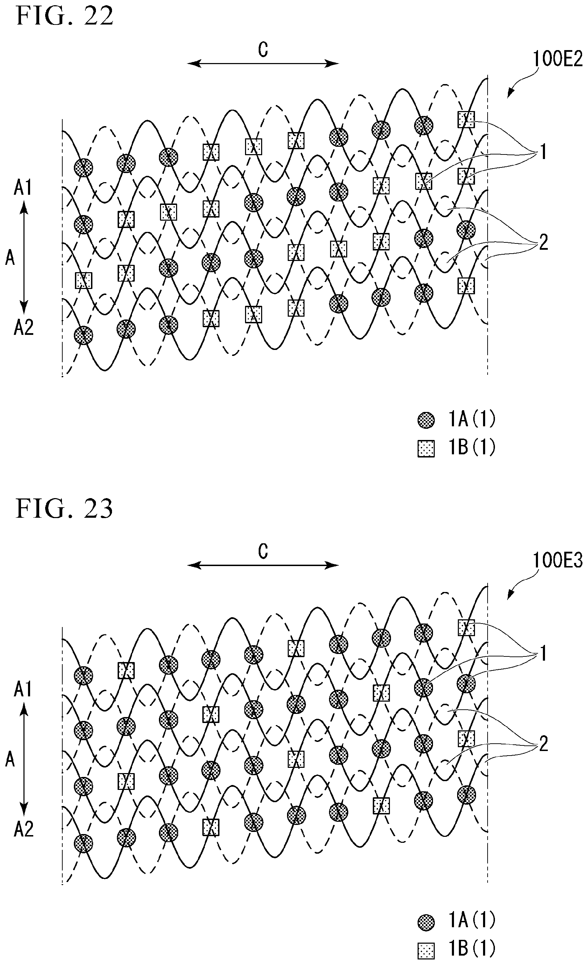

FIG. 22 is a diagram showing a stent 100E2 that is a modification of the arrangement of the first straight-line crossing portion 1A and the second straight-line crossing portion 1B. The three first straight-line crossing portions 1A continuous in the circumferential direction C are arranged spirally along the longitudinal axis direction A. Further, three second straight-line crossing portions 1B continuous in the circumferential direction C are arranged spirally along the longitudinal axis direction A.

### (Modification 5-3)

FIG. 23 is a diagram showing a stent 100E3 that is a modification of the arrangement of the first straight-line crossing portion 1A and the second straight-line crossing portion 1B. The three first straight-line crossing portions 1A continuous in the circumferential direction C are arranged spirally along the longitudinal axis direction A. Moreover, one second straight-line crossing portion 1B continuous in the circumferential direction C is arranged spirally along the longitudinal axis direction A.

### (Modification 5-4)

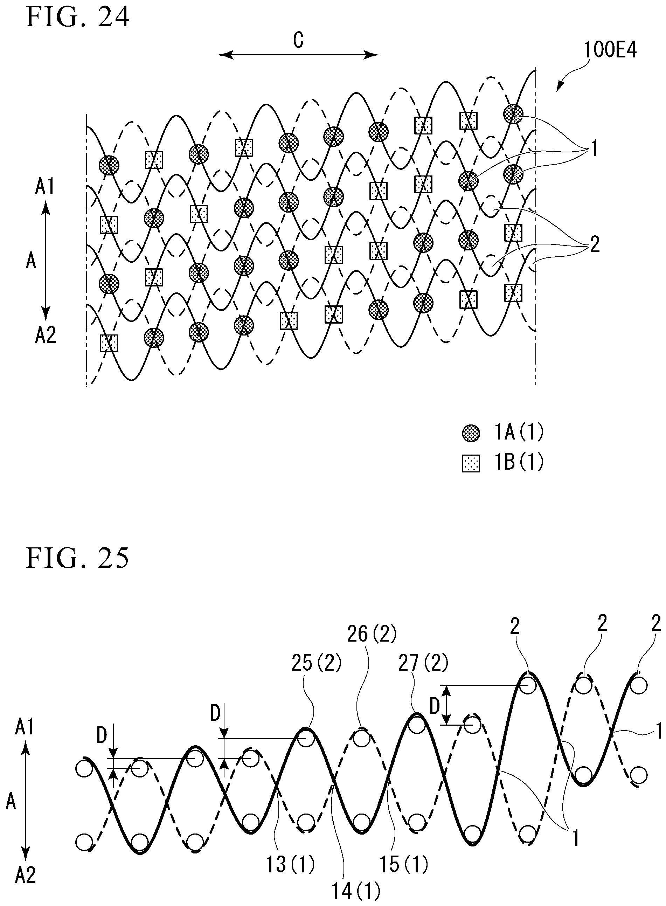

FIG. 24 is a diagram showing a stent 100E4 that is a modification of the arrangement of the first straight-line crossing portion 1A and the second straight-line crossing portion 1B. Three or two first straight-line crossing portions 1A continuous in the circumferential direction C are arranged spirally along the longitudinal axis direction A. Two second straight-line crossing portions 1B continuous in the circumferential direction C are arranged spirally along the longitudinal axis direction A.

### (Modification 5-5)

In the above embodiment, the plurality of interlocking portions 2 are spirally arranged along the longitudinal axis direction A. The distance D in the longitudinal axis direction A between the interlocking portions 2 adjacent in the circumferential direction C is substantially the same. However, the arrangement mode of the plurality of interlocking portions 2 is not limited to this. FIGS. 25A and 25B are diagrams showing a modification of the arrangement of the plurality of interlocking portions 2. The distance D in the longitudinal direction A between the interlocking portions 2 adjacent to each other in the circumferential direction C does not have to be a constant distance. Moreover, the plurality of interlocking portions 2 may be arranged in a spiral shape as a whole. The same is true for the plurality of straight-line crossing portions 1.

As shown in FIG. 25, the plurality of interlocking portions 2 include a fifth interlocking portion 25, a sixth interlocking portion 26, and a seventh interlocking portion 27. The sixth interlocking portion 26 is adjacent to the fifth interlocking portion 25 in the circumferential direction C. The seventh interlocking portion 27 is adjacent to the sixth interlocking portion 26 in the circumferential direction C. The positions of the fifth interlocking portion 25 and the sixth interlocking portion 26 in the longitudinal direction A are substantially the same, and the positions of the sixth interlocking portion 26 and the seventh interlocking portion 27 in the longitudinal direction A are different.

As shown in FIG. 25, the plurality of straight-line crossing portions 1 include a third straight-line crossing portion 13, a fourth straight-line crossing portion 1 F4, and a fifth straight-line crossing portion 15. The fourth straight-line crossing portion 14 is adjacent to the third straight-line crossing portion 13 in the circumferential direction C. The fifth straight-line crossing portion 15 is adjacent to the fourth straight-line crossing portion 14 in the circumferential direction C. The positions of the longitudinal axis direction A of the third straight-line crossing portion 13 and the fourth straight-line crossing portion 14 are substantially the same, and the positions of the longitudinal axis direction A of the fourth straight-line crossing portion 14 and the fifth straight-line crossing portion 15 are different.

### (Sixth embodiment)

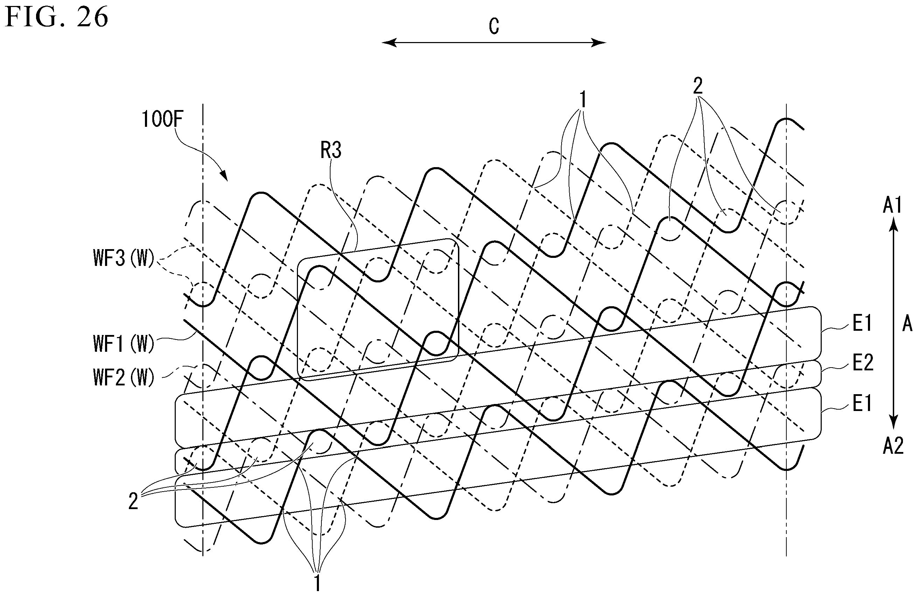

A sixth embodiment of the present invention will be described with reference to FIGS. 26 to 27. In the following description, the same reference numerals are given to the same configurations as those already described, and redundant descriptions will be omitted. A stent 100F according to the sixth embodiment is housed in a stent delivery system 150, like the stent 100 according to the first embodiment.

FIG. 26 is a developed view of the stent 100F deployed in the circumferential direction C.

The stent 100F is formed in the shape of a circular tube having meshes on its peripheral surface by means of wires W that extend obliquely in the circumferential direction C while repeatedly bending. The stent 100F has a plurality of straight-line crossing portions 1 and a plurality of the interlocking portions 2.

As shown in FIG. 26, first regions E1 in which the plurality of straight-line crossing portions 1 are arranged and second regions E2 in which the plurality of interlocking portions 2 are arranged are alternately arranged in the longitudinal axis direction A. The first region E1 is spirally arranged along the longitudinal axis direction A. In addition, the second region E2 is spirally arranged along the longitudinal axis direction A.

In the end region of the stent 100F on the second direction A2 side, the ends of the valley-shaped bent portion 4 on the second direction A2 side are arranged spirally without crossing the peak-shaped bent portion 3, as shown in FIG. 26, or may be aligned with each other in the longitudinal axis direction A. The same applies to the ends of the peak-shaped bent portions 3 on the first direction A1 side in the end regions of the stent 100F on the first direction A1 side. For example, as in the end regions of the stent 100 shown in FIG. 34 and the stent 100K shown in FIG. 38. Thus, the positions of the ends with respect to the longitudinal axis direction A can be aligned.

### [First straight-line crossing portion 1F1]

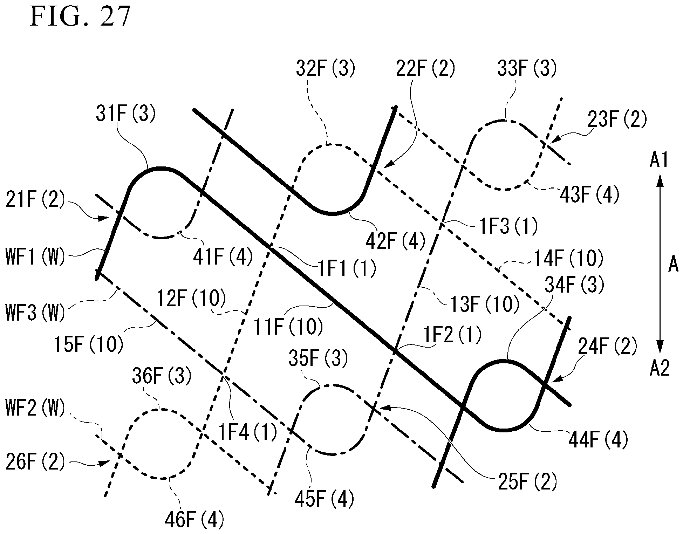

FIG. 27 is an enlarged view of region R3 shown in FIG. 26.

A first straight-line portion 11F and a second straight-line portion 12F, which are the straight-line portions 10, intersect at the "first straight-line crossing portion 1F1", which is the straight-line crossing portion 1, when viewed from the radial direction R of the stent 100F.

### [Second Straight-line crossing portion 1F2]

The first straight-line portion 11F and the third straight-line portion 13F, which are the straight-line portions 10, intersect at the "second straight-line crossing portion 1F2", which is the straight-line crossing portion 1, when viewed from the radial direction R of the stent 100F. The second straight-line crossing portion 1F2 is arranged on the second direction A2 side with respect to the first straight-line crossing portion 1F1.

### [Third Straight-line crossing portion 1F3]

The third straight-line portion 13F and the fourth straight-line portion 14F, which are the straight-line portions 10, intersect at the "third straight-line crossing portion 1F3", which is the straight-line crossing portion 1, when viewed from the radial direction R of the stent 100F. The third straight-line crossing portion 1F3 is arranged on the first direction A1 side with respect to the second straight-line crossing portion 1F2.

### [Fourth Straight-line crossing portion 1F4]

The second straight-line portion 12F and the fifth straight-line portion 15F, which are the straight-line portions 10, intersect at the "fourth straight-line crossing portion 1F4", which is the straight-line crossing portion 1, when viewed from the radial direction R of the stent 100F. The fourth straight-line crossing portion 1F4 is arranged on the second direction A2 side with respect to the first straight-line crossing portion 1F1.

The first straight-line crossing portion 1F1, the second straight-line crossing portion 1F2, the third straight-line crossing portion 1F3, and the fourth straight-line crossing portion 1F4 are arranged in the same first region E1.

### [First interlocking portion 21F]

A "first peak 31F", which is the peak-shaped bent portion 3, is connected to the first direction A1 side of the first straight-line portion 11F. The first peak 31F intersects with the first valley 41F, which is the valley-shaped bent portion 4, to form the "first interlocking portion (upper interlocking portion) 21F", which is the interlocking portion 2.

### [Second interlocking portion 22F]

The "second peak 32F", which is the peak-shaped bent portion 3, is connected to the first direction A1 side of the second straight-line portion 12F and the fourth straight-line portion 14F. The second peak 32F intersects with the second valley 42F, which is the valley-shaped bent portion 4, to form the "second interlocking portion 22F", which is the interlocking portion 2.

### [Third interlocking portion 23F]

The "third peak 33F", which is the peak-shaped bent portion 3, continues on the first direction A1 side of the third straight-line portion 13F. The third peak 33F intersects with the third valley 43F, which is the valley-shaped bent portion 4, to form the "third interlocking portion 23F", which is the interlocking portion 2.

### [Fourth interlocking portion 24F]

The "fourth valley 44F", which is the valley-shaped bent part 4, is connected to the second direction A2 side of the first straight-line portion 11F. The fourth valley 44F intersects with the fourth peak 34F, which is the peak-shaped bent portion 3, to form the "fourth interlocking portion (lower interlocking portion) 24F", which is the interlocking portion 2.

### [Fifth interlocking portion 25F]

The "fifth valley 45F", which is the valley-shaped bent part 4, continues on the second direction A2 side of the third straight-line portion 13F and the fifth straight-line portion 15F. The fifth valley 45F intersects with the fifth peak 35F, which is the peak-shaped bent portion 3, to form the "fifth hook portion 25F", which is the hook portion 2.

### [Sixth interlocking portion 26F]

A "sixth valley 46F", which is the valley-shaped bent part 4, continues on the second direction A2 side of the second straight-line portion 12F. The sixth valley 46F intersects with the sixth peak 36F, which is the peak-shaped bent portion 3, to form the "sixth interlocking portion 26F", which is the interlocking portion 2.

### [Crossing portion of first straight-line portion 11F]

The first straight-line portion 11F is connected to the first interlocking portion (upper interlocking portion) 21F on the first direction A1 side and to the fourth interlocking portion (lower interlocking portion) 24F on the second direction A2 side. The first straight-line portion 11F is located between the first interlocking portion (upper interlocking portion) 21F and the fourth interlocking portion (lower interlocking portion) 24F, and the other two straight-line portions 10 (the second straight-line portion 12F, the third 13F) and straight-line crossing portions 1 (first straight-line crossing portion 1F1, second straight-line crossing portion 1F2).

### [Arrangement of first interlocking portion 21F, second interlocking portion 22F, and third interlocking portion 23F]

The first interlocking portion 21F, the second interlocking portion 22F, and the third interlocking portion 23F are arranged along the circumferential direction C and arranged in the same second region E2.

The first interlocking portion 21F and the second interlocking portion 22F are adjacent to each other in the circumferential direction C and arranged at different positions in the longitudinal direction A. Specifically, the second interlocking portion 22F is arranged on the first direction A1 side in the longitudinal axis direction A from the first interlocking portion 21F.

The second interlocking portion 22F and the third interlocking portion 23F are adjacent to each other in the circumferential direction C and arranged at different positions in the longitudinal direction A. Specifically, the third interlocking portion 23F is arranged on the first direction A1 side in the longitudinal axis direction A from the second interlocking portion 22F.

### [Arrangement of fourth interlocking portion 24F, fifth interlocking portion 25F, and sixth interlocking portion 26F]

The fourth interlocking portion 24F, the fifth interlocking portion 25F, and the sixth interlocking portion 26F are arranged along the circumferential direction C and arranged in the same second region E2.

The fourth interlocking portion 24F and the fifth interlocking portion 25F are adjacent to each other in the circumferential direction C and arranged at different positions in the longitudinal direction A. Specifically, the fifth interlocking portion 25F is arranged on the second direction A2 side in the longitudinal axis direction A from the fourth interlocking portion 24F.

The fifth interlocking portion 25F and the sixth interlocking portion 26F are adjacent to each other in the circumferential direction C and arranged at different positions in the longitudinal direction A. Specifically, the sixth interlocking portion 26F is arranged on the second direction A2 side in the longitudinal axis direction A from the fifth interlocking portion 25F.

### [Wire W]

The first peak 31F, the first straight-line portion 11F, and the fourth valley 44F are a continuous part of the wire W extending in a zigzag along the circumferential direction C (first wire WF1). The sixth valley 46F, the second straight-line portion 12F, the second peak 32F, and the fourth straight-line portion 14F are continuous parts of the wire W extending in a zigzag along the circumferential direction C (second wire WF2). Further, the fifth straight-line portion 15F, the fifth valley 45F, the third straight-line portion 13F, and the third peak 33F are continuous parts (third wire WF3) of the wire W extending in a zigzag along the circumferential direction C. The first wire WF1, the second wire WF2 and the third wire WF3 may be one continuous wire or may be different wires.

In this embodiment, the second valley 42F and the fourth peak 34F are formed by the first wire WF1, the third valley 43F and the sixth peak 36F are formed by the second wire WF2, and the first valley 41F and the fifth peak 35F are formed by the third wire WF3.

### [Other straight-line crossing portions 1 and interlocking portions 2]

The straight-line crossing portion 1 (first straight-line crossing portion 1F1, second straight-line crossing portion 1F2, third straight-line crossing portion 1F3 and fourth straight-line crossing portion 1F4), and six interlocking portions 2 (the first interlocking portion 21F, the second interlocking portion 22F, the third interlocking portion 23F, the fourth interlocking portion 24F, the fifth interlocking portion 25F and the sixth interlocking portion 26F) have the above configuration. As shown in FIG. 26, the interlocking portion 2 connected to another straight-line crossing portion 1 in the stent 100F has the same configuration as the above configuration.

The stent 100F of the present embodiment includes a plurality of interlocking portions 2, and has high shape followability even when bent. In the stent 100F, since it is not necessary to arrange the straight-line crossing portion 1 at a position adjacent to the interlocking portion 2 in the circumferential direction C, the force (axial force) against bending is reduced. Furthermore, since the stent 100F has more straight-line crossing portions 1 than the stent 100 of the first embodiment, friction of the strut is likely to occur between the straight-line crossing portion 1 and the interlocking portion 2 when the entire stent is bent, and the shape retention is high.

The sixth embodiment of the present invention has been described above in detail with reference to the drawings, but the specific configuration is not limited to this embodiment, and design changes and the like are included within the scope of the present invention. Also, the constituent elements shown in the above-described embodiment and modifications shown below can be combined as appropriate.

### (Seventh embodiment)

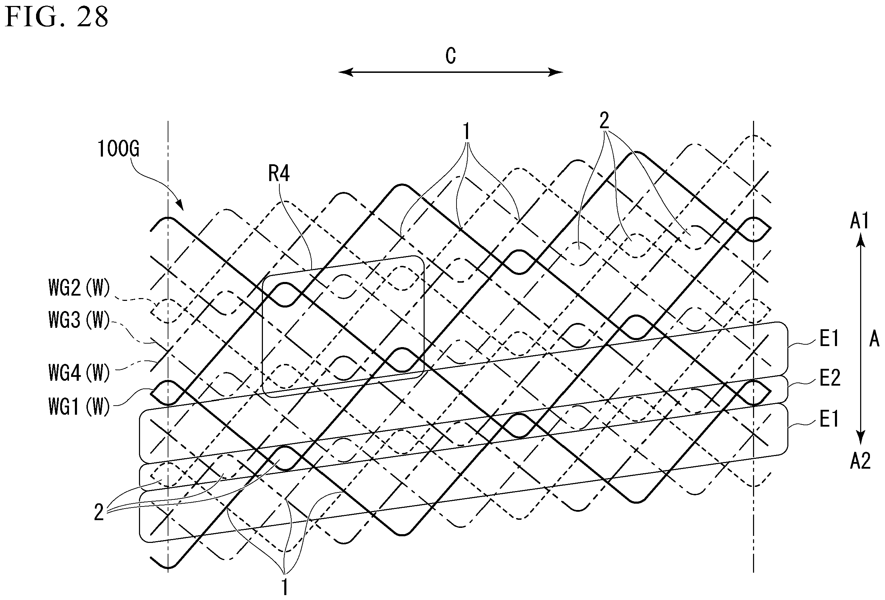

A seventh embodiment of the present invention will be described with reference to FIGS. 28 to 29. In the following description, the same reference numerals are given to the same configurations as those already described, and redundant descriptions will be omitted. A stent 100G according to the seventh embodiment is accommodated in a stent delivery system 150, like the stent 100 according to the first embodiment.

FIG. 28 is a developed view of the stent 100G deployed in the circumferential direction C.

The stent 100G is formed in the shape of a circular tube having meshes on its peripheral surface by means of wires W that extend obliquely in the circumferential direction C while repeating bending. The stent 100G has a plurality of straight-line crossing portions 1 and a plurality of interlocking portions 2.

As shown in FIG. 28, the first regions E1 in which the plurality of straight-line crossing portions 1 are arranged and the second regions E2 in which the plurality of interlocking portions 2 are arranged are alternately arranged in the longitudinal axis direction A. The first region E1 is spirally arranged along the longitudinal axis direction A. In addition, the second region E2 is spirally arranged along the longitudinal axis direction A.

In the end region of the stent 100G on the second direction A2 side, the ends of the valley-shaped bent portion 4 on the second direction A2 side are arranged in a spiral shape without crossing the peak-shaped bent portion 3, as shown in FIG. 28, or may be aligned with each other in the longitudinal axis direction A. The same applies to the ends of the peak-shaped bent portions 3 on the first direction A1 side in the end regions of the stent 100G on the first direction A1 side. For example, as in the end regions of the stent 100 shown in FIG. 34 and the stent 100K shown in FIG. 38. Thus, the positions of the ends with respect to the longitudinal axis direction A can be aligned.

### [First straight-line crossing portion 1G1]

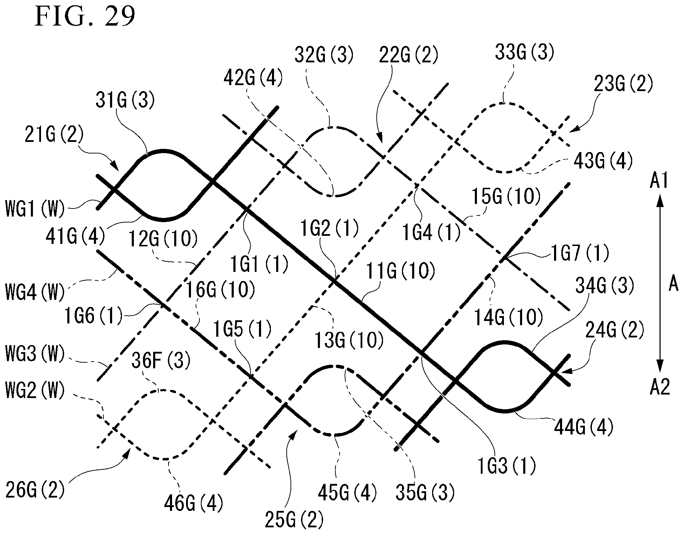

FIG. 29 is an enlarged view of region R4 shown in FIG. 28.

The first straight-line portion 11G and the second straight-line portion 12G, which are the straight-line portions 10, intersect at the "first straight-line crossing portion 1G1", which is the straight-line crossing portion 1, when viewed from the radial direction R of the stent 100G.

### [Second Straight-line crossing portion 1G2]

The first straight-line portion 11G and the third straight-line portion 13G, which are the straight-line portions 10, intersect at the "second straight-line crossing portion 1G2", which is the straight-line crossing portion 1, when viewed from the radial direction R of the stent 100G. The second straight-line crossing portion 1G2 is arranged on the second direction A2 side with respect to the first straight-line crossing portion 1G1.

### [Third Straight-line crossing portion 1G3]

The first straight-line portion 11G and the fourth straight-line portion 14G, which are the straight-line portions 10, intersect at the "third straight-line crossing portion 1G3", which is the straight-line crossing portion 1, when viewed from the radial direction R of the stent 100G. The third straight-line crossing portion 1G3 is arranged on the second direction A2 side with respect to the second straight-line crossing portion 1G2.

### [Fourth Straight-line crossing portion 1G4]

The third straight-line portion 13G and the fifth straight-line portion 15G, which are the straight-line portions 10, intersect at the "fourth straight-line crossing portion 1G4", which is the straight-line crossing portion 1, when viewed from the radial direction R of the stent 100G. The fourth straight-line crossing portion 1G4 is arranged on the first direction A1 side with respect to the second straight-line crossing portion 1G2.

### [Fifth Straight-line crossing portion 1G5]

The third straight-line portion 13G and the sixth straight-line portion 16G, which are the straight-line portions 10, intersect at the "fifth straight-line crossing portion 1G5", which is the straight-line crossing portion 1, when viewed from the radial direction R of the stent 100G. The fifth straight-line crossing portion 1G5 is arranged on the second direction A2 side with respect to the second straight-line crossing portion 1G2.

### [Sixth straight-line crossing portion 1G6]

The second straight-line portion 12G and the sixth straight-line portion 16G, which are the straight-line portions 10, intersect at the "sixth straight-line crossing portion 1G6", which is the straight-line crossing portion 1, when viewed from the radial direction R of the stent 100G. The sixth straight-line crossing portion 1G6 is arranged on the second direction A2 side with respect to the first straight-line crossing portion 1G1 and on the first direction A1 side with respect to the fifth straight-line crossing portion 1G5.

### [Seventh Straight-line crossing portion 1G7]

The fourth straight-line portion 14G and the fifth straight-line portion 15G, which are the straight-line portions 10, intersect at the "seventh straight-line crossing portion 1G7", which is the straight-line crossing portion 1, when viewed from the radial direction R of the stent 100G. The seventh straight-line crossing portion 1G7 is arranged on the second direction A2 side with respect to the fourth straight-line crossing portion 1G4 and on the first direction A1 side with respect to the third straight-line crossing portion 1G3.

The first straight-line crossing portion 1G1, the second straight-line crossing portion 1G2, the third straight-line crossing portion 1G3, the fourth straight-line crossing portion 1G4, the fifth straight-line crossing portion 1G5, the sixth straight-line crossing portion 1G6 and the seventh straight-line crossing portion 1G7 are arranged in the same first region E1.

### [First interlocking portion 21G]

A "first peak 31G", which is the peak-shaped bent portion 3, is connected to the first direction A1 side of the first straight-line portion 11G. The first peak 31G intersects with the first valley 41G, which is the valley-shaped bent portion 4, to form the "first interlocking portion (upper interlocking portion) 21G", which is the interlocking portion 2.

### [Second interlocking portion 22G]

A "second peak 32G", which is the peak-shaped bent portion 3, is connected to the first direction A1 side of the second straight-line portion 12G and the fifth straight-line portion 15G. The second peak 32G intersects with the second valley 42G, which is the valley-shaped bent portion 4, to form the "second interlocking portion 22G", which is the interlocking portion 2.

### [Third interlocking portion 23G]

A "third peak 33G", which is the peak-shaped bent portion 3, is connected to the first direction A1 side of the third straight-line portion 13G. The third peak 33G intersects with the third valley 43G, which is the valley-shaped bent portion 4, to form the "third interlocking portion 23G", which is the interlocking portion 2.

### [Fourth interlocking portion 24G]

The "fourth valley 44G", which is the valley-shaped bent part 4, is connected to the second direction A2 side of the first straight-line portion 11G. The fourth valley 44G intersects with the fourth peak 34G, which is the peak-shaped bent portion 3, to form the "fourth interlocking portion (lower interlocking portion) 24G", which is the engaging section 2.

### [Fifth interlocking portion 25G]

A "fifth valley 45G", which is the valley-shaped bent part 4, continues on the second direction A2 side of the fourth straight-line portion 14G and the sixth straight-line portion 16G. The fifth valley 45G intersects with the fifth peak 35G, which is the peak-shaped bent portion 3, to form the "fifth hook portion 25G", which is the hook portion 2.

### [Sixth interlocking portion 26G]

A "sixth valley 46G", which is the valley-shaped bent part 4, continues on the second direction A2 side of the third straight-line portion 13G. The sixth valley 46G intersects with the sixth peak 36G, which is the peak-shaped bent portion 3, to form the "sixth interlocking portion 26G", which is the interlocking portion 2.

### [Crossing portion of first straight-line portion 11G]

The first straight-line portion 11G connects with the first interlocking portion (upper interlocking portion) 21G on the first direction A1 side, and connects with the fourth interlocking portion (lower interlocking portion) 24G on the second direction A2 side. Between the first interlocking portion (upper interlocking portion) 21G and the fourth interlocking portion (lower interlocking portion) 24G, the first straight-line portion 11G and the other three straight-line portions 10 (the second straight-line portion 12G, the third straight-line portion 13G, fourth straight-line portion 14G) constitute the straight-line crossing portions 1 (first straight-line crossing portion 1G1, second straight-line crossing portion 1G2, third straight-line crossing portion 1G3), respectively.

### [Arrangement of first interlocking portion 21G, second interlocking portion 22G, and third interlocking portion 23G]

The first interlocking portion 21G, the second interlocking portion 22G, and the third interlocking portion 23G are arranged along the circumferential direction C and arranged in the same second region E2.

The first interlocking portion 21G and the second interlocking portion 22G are adjacent to each other in the circumferential direction C and arranged at different positions in the longitudinal direction A. Specifically, the second interlocking portion 22G is arranged on the first direction A1 side in the longitudinal axis direction A from the first interlocking portion 21G.

The second interlocking portion 22G and the third interlocking portion 23G are adjacent to each other in the circumferential direction C and arranged at different positions in the longitudinal direction A. Specifically, the third interlocking portion 23G is arranged on the first direction A1 side in the longitudinal axis direction A from the second interlocking portion 22G.

### [Arrangement of fourth interlocking portion 24G, fifth interlocking portion 25G, and sixth interlocking portion 26G]

The fourth interlocking portion 24G, the fifth interlocking portion 25G, and the sixth interlocking portion 26G are arranged along the circumferential direction C and arranged in the same second region E2.

The fourth interlocking portion 24G and the fifth interlocking portion 25G are adjacent to each other in the circumferential direction C and arranged at different positions in the longitudinal direction A. Specifically, the fifth interlocking portion 25G is arranged on the second direction A2 side in the longitudinal axis direction A from the fourth interlocking portion 24G.

The fifth interlocking portion 25G and the sixth interlocking portion 26G are adjacent to each other in the circumferential direction C and arranged at different positions in the longitudinal direction A. Specifically, the sixth interlocking portion 26G is arranged on the second direction A2 side in the longitudinal axis direction A from the fifth interlocking portion 25G.

### [Wire W]

The first peak 31G, the first straight-line portion 11G, and the fourth valley 44G are a continuous part of the wire W extending in a zigzag along the circumferential direction C (first wire WG1). Also, the sixth valley 46G, the third straight-line portion 13G, and the third peak 33G are a continuous part of the wire W extending in a zigzag along the circumferential direction C (second wire WG2). Further, the second straight-line portion 12G, the second peak 32G, and the fifth straight-line portion 15G are continuous parts of the wire W extending in a zigzag along the circumferential direction C (third wire WG3). The sixth straight-line portion 16G, the fifth valley 45G, and the fourth straight-line portion 14G are continuous portions of the wire W extending in a zigzag along the circumferential direction C (fourth wire WG4). The first wire WG1, the second wire WG2, the third wire WG3, and the fourth wire WG4 may be one continuous wire, or may be different wires.

In this embodiment, the first valley 41G and the fourth peak 34G are formed by the first wire WG1, the third valley 43G and the sixth peak 36G are formed by the second wire WG2, the second valley 42G is formed by the third wire WG3, and the fifth peak 35G is formed by the fourth wire WG4.

### [Other straight-line crossing portions 1 and interlocking portions 2]

The straight-line crossing portion 1 (first straight-line crossing portion 1G1, second straight-line crossing portion 1G2, third straight-line crossing portion 1G3, fourth straight-line crossing portion 1G4, fifth straight-line crossing portion 1G5, sixth straight-line crossing portion 1G6 and seventh straight-line crossing portion 1G7) and the six interlocking portions 2 (first interlocking portion 21G, second interlocking portion 22G, third interlocking portion 23G, fourth interlocking portion 24G, fifth interlocking portion 25G and sixth interlocking portion 26G) connected to the straight-line crossing portion 1 are configured as described above. As shown in FIG. 28, the interlocking portion 2 connected to another straight-line crossing portion 1 in the stent 100G has the same configuration as the above configuration.

According to the stent 100G of the present embodiment, it has a plurality of interlocking portions 2 and has high shape followability even when bent. In the stent 100G, since it is not necessary to arrange the straight-line crossing portion 1 at a position adjacent to the interlocking portion 2 in the circumferential direction C, the force (axial force) against bending is reduced. Furthermore, since the stent 100G has more straight-line crossing portions 1 than the stent 100F of the sixth embodiment, when the stent as a whole is bent, strut friction is likely to occur between the linear crossing portion 1 and the interlocking portion 2, resulting in higher shape retention.

The seventh embodiment of the present invention has been described above in detail with reference to the drawings, but the specific configuration is not limited to this embodiment, and design changes and the like are included within the scope of the present invention. Also, the constituent elements shown in the above-described embodiment and modifications shown below can be combined as appropriate.

### (Modification 7-1)

In the sixth embodiment, between the first interlocking portion (upper interlocking portion) 21F and the fourth interlocking portion (lower interlocking portion) 24F, the first straight-line portion 11F and the other two straight-line portions 10 (the second straight-line portion 12F and the third straight-line portion 13F) constitute the straight-line crossing portions 1 (the first straight-line crossing portion 1F 1 and the second straight-line crossing portion 1F2) respectively. In the seventh embodiment, between the first interlocking portion (upper interlocking portion) 21G and the fourth interlocking portion (lower interlocking portion) 24G, the first straight line portion 11G and the other three straight line portions 10 (second straight-line portion 12G, third straight-line portion 13G, fourth straight-line portion 14G) respectively constitute the straight-line portion portions 1 (the first straight-line portion 1G1, the second straight-line portion 1G2, and the third straight-line portion 1G3). However, the aspect of the first straight-line portion is not limited to this. The first straight-line portion may form the straight-line crossing portions 1 with four or more other straight-line portions 10 between the first interlocking portion (upper interlocking portion) and the fourth interlocking portion (lower interlocking portion).

### (Modification 7-2)

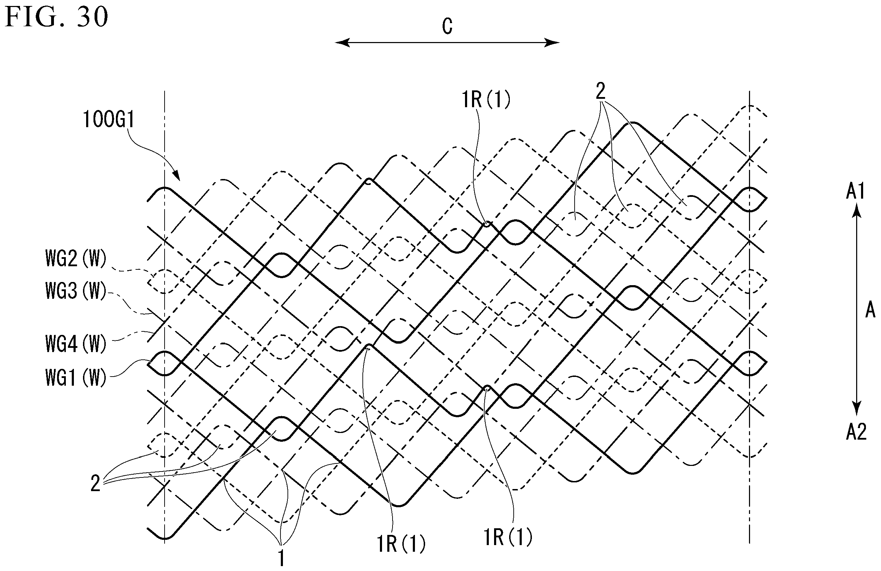

FIG. 30 is an exploded view of a stent 100G1 that is a modification of the stent 100G.

In the stent 100G1, the straight-line crossing portion 1 of the stent 100G is replaced with an interlocking portion 2 (hereinafter also referred to as "replacement interlocking portion 1R"). For example, by changing the routes of the first wire WG1 and the fourth wire WG4 as shown in FIG. 30, a part of the straight-line crossing portion 1 can be replaced with the replacement interlocking portion 1R.

In the end region of the stent 100G1 on the second direction A2 side, the ends of the valley-shaped bent portion 4 on the second direction A2 side are arranged in a spiral shape without crossing the peak-shaped bent portion 3 as shown in FIG. 30, or may be aligned with each other in the longitudinal axis direction A. In addition, the same applies to the ends of the peak-shaped bent portions 3 on the first direction A1 side in the end regions of the stent 100G1 on the first direction A1 side. For example, as in the end regions of the stent 100 shown in FIG. 34 and the stent 100K shown in FIG. 38. Thus, the positions of the ends with respect to the longitudinal axis direction A can be aligned.

### (Eighth embodiment)

An eighth embodiment of the present invention will be described with reference to FIG. In the following description, the same reference numerals are given to the same configurations as those already described, and redundant descriptions will be omitted. A stent 100H according to the eighth embodiment is housed in a stent delivery system 150, like the stent 100 according to the first embodiment.

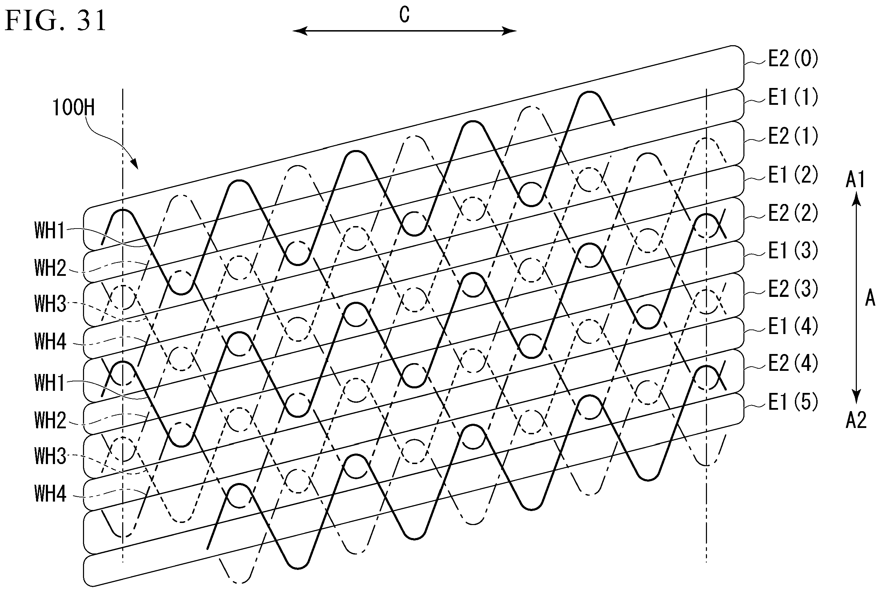

FIG. 31 is a developed view of the stent 100H deployed in the circumferential direction C.

The stent 100H is formed in the shape of a circular tube having meshes on its peripheral surface by means of wires W that extend obliquely in the circumferential direction C while repeatedly bending. The stent 100H has a plurality of straight-line crossing portions 1 and a plurality of interlocking portions 2.

As shown in FIG. 31, the first regions E1 in which the plurality of straight-line crossing portions 1 are arranged and the second regions E2 in which the plurality of interlocking portions 2 are arranged are alternately arranged in the longitudinal axis direction A. The first region E1 is spirally arranged along the longitudinal axis direction A. In addition, the second region E2 is spirally arranged along the longitudinal axis direction A.

As shown in FIG. 31, in the state of being developed in the circumferential direction C, the first region E1 and the second region E2, which are alternately arranged along the longitudinal axis direction A toward the second direction A2, are distinguished as second region E2(0), first region E1(1), second region E2(1), first region E1(2), second region E2(2), first region E1(3), second region E2(3), first region E1(4) and a second region E2(4). On both sides of the longitudinal axis direction A across the second region E2(n), the first region E1(n) is arranged on the first direction A1 side, and the first region E1(n+1) is arranged on the second direction A2 side, wherein n is an integer.

In the first region E1(1), a straight-line crossing portion 1 is formed by two wires W (first wire WH1 and second wire WH2), similar to the stent 100 of the first embodiment.

In the first region E1 (2), a straight-line crossing portion 1 is formed by two wires W (third wire WH3 and fourth wire WH4), similar to the stent 100 of the first embodiment. The two wires W forming the first region E1(2) are both different from the wires W forming the first region E1(1).

In the first region E1 (3), a straight-line crossing portion 1 is formed by two wires W (first wire WH1 and second wire WH2), similar to the stent 100 of the first embodiment. The two wires W forming the first region E1(3) are both the same as the wires W forming the first region E1(1).

In the first region E1 (4), a straight-line crossing portion 1 is formed by two wires W (third wire WH3 and fourth wire WH4), like the stent 100 of the first embodiment. The two wires W forming the first region E1(4) are both the same as the wires W forming the first region E1(2).

That is, the first regions E1 arranged on both sides in the longitudinal direction A with the first region E2 interposed therebetween are formed of different wires W. In this embodiment, the first regions E1 formed of different wire groups W are alternately arranged in the longitudinal axis direction A.

In the first direction A1 side end region of the stent 100H, the ends of the peak-shaped bent portion 3 on the first direction A1 side may be spirally arranged without intersecting the valley-shaped bent portion 4 as in the E2(0) region of FIG. 31, or may be arranged so as to align their positions with respect to the longitudinal axis direction A. The same applies to the second direction A2 side end portion of the valley-shaped bent portion 4 in the second direction A2 side end portion region of the stent 100H. For example, as in the end regions of the stent 100 shown in FIG. 34 and the stent 100K shown in FIG. 38, by adjusting the lengths in the longitudinal direction A of the peak-shaped bent portion 3 and the valley-shaped bent portion 4 in the end region, the positions of the ends with respect to the longitudinal direction A can be aligned. Specifically, when there are two wire groups as in this embodiment, the ends of one end region of stent 100H can be aligned by adjusting the length of one wire group. Also, the ends of the other end region of stent 100H can be aligned by adjusting the length of the other wire group.

According to the stent 100H of the present embodiment, it has a plurality of interlocking portions 2 and has a high shape followability even when it is bent. Since the stent 100H does not need to arrange the straight-line crossing portion 1 at a position adjacent to the interlocking portion 2 in the circumferential direction C, the force (axial force) against bending is reduced.

The eighth embodiment of the present invention has been described above in detail with reference to the drawings, but the specific configuration is not limited to this embodiment, and design changes and the like are also included within the scope of the present invention. Also, the constituent elements shown in the above-described embodiment and modifications shown below can be combined as appropriate.

### (Modification 8-1)

In the above embodiment, two types of first regions E1 formed by different wire groups W are alternately arranged in the longitudinal axis direction A. However, the arrangement mode of the first regions E1 formed by different wire groups W is not limited to this. Three or more types of first regions E1 formed of different wire groups W may be alternately arranged in the longitudinal axis direction A. Two or more types of first regions E1 formed of different wire groups W may be arranged irregularly in the longitudinal axis direction A.

### (Ninth embodiment)

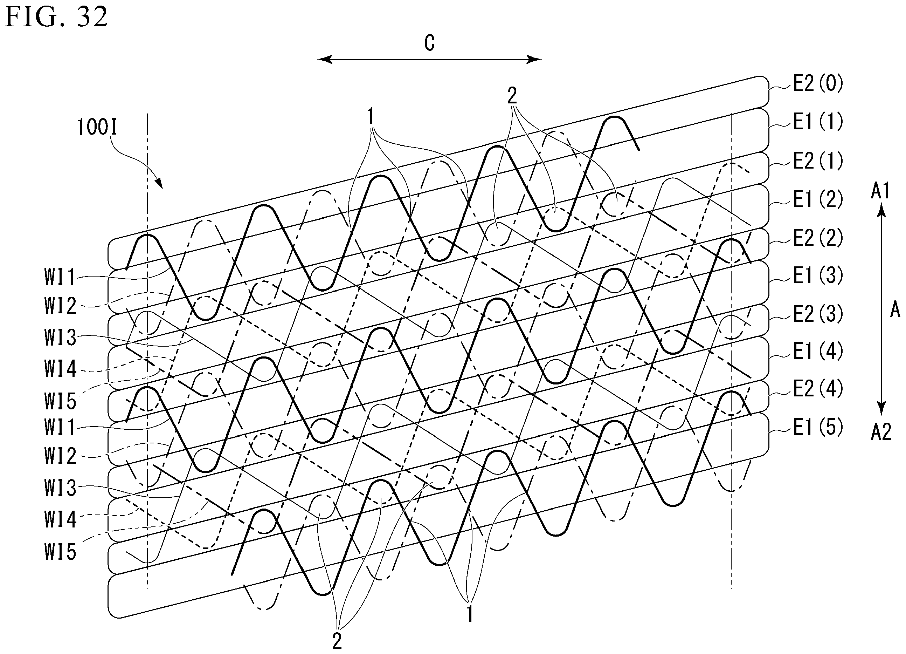

A ninth embodiment of the present invention will be described with reference to FIG. 32. In the following description, the same reference numerals are given to the same configurations as those already described, and redundant descriptions will be omitted. A stent 100I according to the ninth embodiment is housed in a stent delivery system 150, like the stent 100 according to the first embodiment.

FIG. 32 is a developed view of the stent 100I deployed in the circumferential direction C.

The stent 100I is formed in the shape of a circular tube having meshes on its peripheral surface by means of wires W that extend obliquely in the circumferential direction C while repeating bending. The stent 100I has a plurality of straight-line crossing portions 1 and a plurality of interlocking portions 2.

As shown in FIG. 32, first regions E1 in which a plurality of straight-line crossing portions 1 are arranged and second regions E2 in which a plurality of interlocking portions 2 are arranged are alternately arranged in the longitudinal axis direction A. The first region E1 is spirally arranged along the longitudinal axis direction A. In addition, the second region E2 is spirally arranged along the longitudinal axis direction A.

As shown in FIG. 32, in the state of being developed in the circumferential direction C, the first region E1 and the second region E2, which are alternately arranged in the second direction A2 along the longitudinal axis direction A, are distinguished as second region E2 (0), first region E1(1), second region E2(1), first region E1(2), second region E2(2), first region E1(3), second region E2(3), first region E1(4) and second region E2(4). On both sides of the longitudinal axis direction A across the second region E2(n), the first region E1(n) is arranged on the first direction A1 side, and the first region E1(n+1) is arranged on the second direction A2 side, wherein n is an integer.

In the first region E1(1), a straight-line crossing portion 1 is formed by two wires W (first wire WI1 and second wire WI2), like the stent 100 of the first embodiment.

In the first region E1 (2), a straight-line crossing portion 1 is formed by three wires W (the third wire WI3, the second wire WI4, and the fifth wire WI5), as in the stent 100F of the sixth embodiment. The wire W forming the first region E1(2) is different from the wire W forming the first region E1(1). The number (three) of overlapping wires W forming the first region E1(2) is different from the number (two) of overlapping wires W forming the first region E1(1).

In the first region E1(3), a straight-line crossing portion 1 is formed by two wires W (first wire WI1 and second wire WI2), like the stent 100 of the first embodiment. The two wires W forming the first region E1(3) are both the same as the wires W forming the first region E1(1). The number (two) of overlapping wires W forming the first region E1(3) is the same as the number (two) of overlapping wires W forming the first region E1(1).

In the first region E1 (4), a straight-line crossing portion 1 is formed by three wires W (third wire WI3, second wire WI4, and fifth wire WI5) in the same manner as the stent 100F of the sixth embodiment. The three wires W forming the first region E1(4) are the same as the wires W forming the first region E1(2). The number (three) of overlapping wires W forming the first region E1(4) is the same as the number (three) of overlapping wires W forming the first region E1(3).

That is, the first regions E1 arranged on both sides in the longitudinal direction A with the first region E2 interposed therebetween are formed of different wires W and formed by different wire weaving methods. In this embodiment, the first regions E1 formed by different weaving methods of the wires W are alternately arranged in the longitudinal axis direction A.

In the first direction A1 side end region of the stent 100I, the end portions of the first direction A1 side of the peak-shaped bent portion 3 may be arranged in a spiral shape without intersecting with the valley-shaped bent portion 4 as shown in the E2(0) region of FIG. 32, and may be arranged so as to align their positions with respect to the longitudinal axis direction A. The same applies to the second direction A2 side end portion of the valley-shaped bent portion 4 in the second direction A2 side end portion region of the stent 100I. For example, as in the end regions of the stent 100 shown in FIG. 34 and the stent 100K shown in FIG. 38, by adjusting the lengths in the longitudinal direction A of the peak-shaped bent portion 3 and the valley-shaped bent portion 4 in the end regions, the positions of the ends with respect to the longitudinal direction A can be aligned. Specifically, when there are two wire groups, as in this embodiment, the ends of one end region of stent 100I can be aligned by adjusting the length of one wire group. Also, the ends of the other end region of stent 100I can be aligned by adjusting the length of the other wire group.

According to the stent 100I of the present embodiment, it has a plurality of interlocking portions 2 and has a high shape followability even when bent. Since the stent 100I does not need to arrange the straight-line crossing portion 1 at a position adjacent to the interlocking portion 2 in the circumferential direction C, the force (axial force) against bending is reduced. Furthermore, the stent 100I can selectively change the shape retention according to the location by changing the weaving method of the wires W for each first region E1. Furthermore, the stent 100I can selectively change the cell size and the like depending on the expansion force and location. For example, stent 100I can have a smaller cell size to better accommodate ingrowth, or a larger cell size to facilitate special procedures such as stent-in-stent.

The ninth embodiment of the present invention has been described above in detail with reference to the drawings, but the specific configuration is not limited to this embodiment, and design changes and the like are included within the scope of the present invention. Also, the constituent elements shown in the above-described embodiment and modifications shown below can be combined as appropriate.

### (Modification 9-1)

In the above embodiment, the two types of first regions E1 formed by different weaving methods of the wires W are alternately arranged in the longitudinal axis direction A. However, the arrangement mode of the first regions E1 formed by different weaving methods of the wires W is not limited to this. Three or more types of first regions E1 formed by different weaving methods of the wires W may be alternately arranged in the longitudinal axis direction A. Two or more types of first regions E1 formed by different weaving methods of the wires W may be arranged irregularly in the longitudinal axis direction A.

### (Tenth embodiment)

A tenth embodiment of the present invention will be described with reference to FIGS. 33 to 34. In the following description, the same reference numerals are given to the same configurations as those already described, and redundant descriptions will be omitted.

The stent manufacturing method of the present embodiment is a method of manufacturing the stent 100 according to the first embodiment, the stent 100B according to the second embodiment, the stent 100C according to the third embodiment, the stent 100D according to the fourth embodiment, and the stent 100E according to the fifth embodiment. Hereinafter, a method for manufacturing the stent 100 according to the first embodiment will be mainly described.

In the stent manufacturing method of this embodiment, the first wire W1 and the second wire W2 (see FIG. 4) forming the stent 100 are two different wires W.

In the stent manufacturing method of this embodiment, a jig as described in US Pat. No. 6,974,472 is used. The jig is formed in a cylindrical shape and has a plurality of projecting pins on its outer periphery. In the jig used in this embodiment, protruding pins are provided at locations where the interlocking portions 2 of the stent 100 are formed, and are spirally arranged along the longitudinal axis direction.

### <First wire weaving step>

FIG. 33 is a developed view of the knitted first wire W1 developed in the circumferential direction C.

An operator weaves the first wire W1 along a cylindrical jig having a plurality of projecting pins spirally provided. The operator weaves the first wire W1 in a zigzag pattern, so as to form a part of the interlocking portion 2 of the second region E2(n-1) and the second region E2(n) on both sides in the longitudinal direction A with the first region E1(n) interposed therebetween.

Specifically, the operator forms the peak-shaped bent portion 3 of the second region E2(n-1) by hooking the first wire W1 on the protruding pin and bending the first wire W1. Next, the operator similarly forms the valley-shaped bent portion 4 of the second region E2(n), which is continuous with the recently formed peak-shaped bent portion 3 via the straight-line portion 10. Next, the operator similarly forms the peak-shaped bent portion 3 of the second region E2(n-1), which is connected to the recently formed valley-shaped bent portion 4 via the other straight-line portion 10. Next, the operator similarly forms valley-shaped bent portions 4 in the second region E2(n), which are connected to the most recently formed peak-shaped bent portions 3 via other straight-line portions 10. Thereafter, the operator repeats this to alternately form the peak-shaped bent portions 3 and the valley-shaped bent portions 4, and knits the first wire W1 along the longitudinal axis direction A.

When the weaving of the first wire W1 is completed, as shown in FIG. 33, the wires W are not overlapped, and neither the straight-line crossing portion 1 nor the interlocking portion 2 is formed.

### <Second wire weaving step>

FIG. 34 is a developed view of the knitted first wire W 1 and second wire W2 developed in the circumferential direction C. The operator weaves the second wire W2 along a columnar jig provided with a plurality of projecting pins spirally. The operator weaves the second wire W2 in a zigzag pattern, so as to form the second region E2(n-1) and the rest of the interlocking portion 2 of the second region E2(n) on both sides in the longitudinal direction A across the first region E1(n).

Specifically, the operator attaches the peak-shaped bent portion 3, which is not formed by the first wire W1 and is in the second region E2(n-1), to the protruding pin with the second wire W2. is hooked to bend the second wire W2. Next, the operator similarly forms the valley-shaped bent portion 4 that is not formed by the first wire W1 and that is the valley-shaped bent portion 4 of the second region E2(n) connected to the immediately formed peak-shaped bent portion 3 via the straight-line portion 10. Next, the operator forms the peak-shaped bent portion 3 in the same manner that is not formed by first wire W1 and that is the peak-shaped bent portion 3 of the second region E2(n-1) that is connected to the valley-shaped bent portion 4 that is formed most recently via another straight portion 10. Next, the operator similarly forms the valley-shaped bent portion 4 that is not formed by the first wire W1 and that is the valley-shaped bent portion 4 of the second region E2(n) connected to the immediately formed peak-shaped bent portion 3 via another straight portion 10. Thereafter, the operator repeats this to alternately form the peak-shaped bent portions 3 and the valley-shaped bent portions 4, and knits the second wire W2 along the longitudinal axis direction A.

When weaving the second wire W2, the operator crosses the first wire W1 with the second wire W2 so that the interlocking portion 2 is formed. The peak-shaped bent portion 3 and the valley-shaped bent portion 4 intersect to form the interlocking portion 2 where the first wire W1 and the second wire W2 intersect. When the second wire W2 is woven, the operator crosses the first wire W1 with the second wire W2 so that the straight-line crossing portion 1 is formed. A straight-line crossing portion 1 is formed by the straight-line portion 10 of the first wire W 1 and the straight-line portion 10 of the second wire W2 crossing each other.

### <Marker mounting step>

The operator attaches X-ray visibility markers to predetermined locations on the first wire W1 and the second wire W2 as necessary.

### <Washing step>

The operator cleans the woven first wire W1 and second wire W2 as necessary.

### <Heat treatment step>

The operator performs heat treatment on the first wire W1 and the second wire W2 that have been woven, and performs shape memory processing on the first wire W1 and the second wire W2. The first wire W1 and the second wire W2 are, for example, a superelastic alloy whose main material is NiTi. A superelastic alloy composed mainly of NiTi is not permanently deformed when it is woven, and the woven shape is memorized by applying a heat treatment in a woven state.

### <Joining step>

The operator joins the end of the first wire W1 and the end of the second wire W2 by caulking, laser welding, tight winding, or the like. The operator may remove the first wire W1 and the second wire W2 from the jig, and then join the end of the first wire W1 and the end of the second wire W2. The operator may join the end of the first wire W1 and the end of the second wire W2 before removing the first wire W1 and the second wire W2 from the jig.

In the stent 100 produced by the stent manufacturing method of this embodiment, an even number of interlocking portions 2 are arranged for each second region E2(n). In the stent 100 illustrated in FIG. 34, twelve interlocking portions 2 are arranged for each second region E2(n). The knitting method of the stent by the stent manufacturing method of the present embodiment is also called "even number knitting".

In the stent 100 produced by the stent manufacturing method of this embodiment, as shown in FIG. 4, the first valleys 41 and the second peaks 32 are formed by the first wire W1. The first peak 31 and the second valley 42 are formed by the second wire W2.

The stent 100B according to the second embodiment, the stent 100C according to the third embodiment, the stent 100D according to the fourth embodiment, and the stent 100E according to the fifth embodiment can also be manufactured by the same manufacturing method.

According to the stent manufacturing method of the present embodiment, it is possible to manufacture the stent 100 or the like, which has a plurality of interlocking portions 2 and has high shape followability even when bent. As shown in FIG. 4, the first valley 41 and the second peak 32 are formed by the first wire W1, and the first peak 31 and the second valley 42 are formed by the second wire W2. Therefore, the stent 100 or the like formed by the stent manufacturing method (even-number knitting) of the present embodiment has a strong skeleton, and can reduce the occurrence of ingrowth and migration.

The tenth embodiment of the present invention has been described above in detail with reference to the drawings, but the specific configuration is not limited to this embodiment, and design changes and the like are included within the scope of the present invention. Also, the constituent elements shown in the above-described embodiment and modifications shown below can be combined as appropriate.

### (Eleventh embodiment)

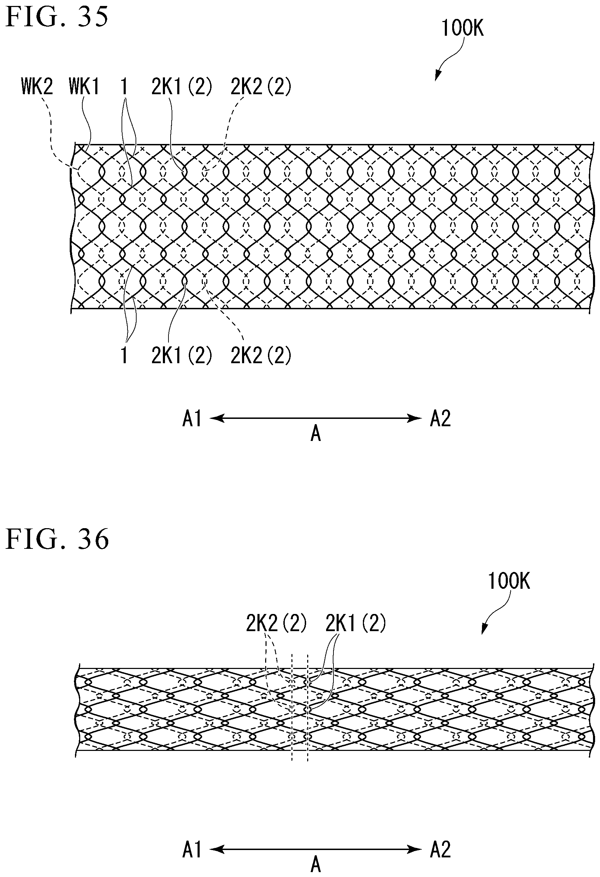

An eleventh embodiment of the present invention will be described with reference to FIGS. 35 to 38. In the following description, the same reference numerals are given to the same configurations as those already described, and redundant descriptions will be omitted.

FIG. 35 is a diagram showing a stent 100K of this embodiment.

The stent 100K differs from the stent 100 according to the first embodiment in the first wire W1 and the second wire W2. The stent 100K is formed by weaving a first wire WK1 and a second wire WK2 different from the first wire WK1.

The stent 100K is formed in the shape of a circular tube having a mesh on the peripheral surface by the first wire WK1 and the second wire WK2 extending at an angle in the circumferential direction C while repeatedly bending. The stent 100K has a plurality of straight-line crossing portions 1 and a plurality of interlocking portions 2.

The straight-line crossing portion 1 of the stent 100K is formed by crossing the straight-line portion 10 of the first wire WK1 and the straight-line portion 10 of the second wire WK2, similarly to the stent 100 according to the first embodiment.

The interlocking portion 2 of the stent 100K differs from the stent 100 according to the first embodiment in that it includes the interlocking portion 2 where the first wires WK1 cross each other (hereinafter also referred to as an interlocking portion 2K1) and the interlocking portion 2 where the second wires WK2 cross each other (hereinafter also referred to as an interlocking portion 2K2). In the stent 100K, the first peaks 31 and the first valleys 41 forming the first interlocking portion 21 shown in FIG. 4 are formed by the first wire WK1. In the stent 100K, the second peaks 32 and the second valleys 42 forming the second interlocking portion 22 shown in FIG. 4 are formed by the second wire WK2.

FIG. 36 is a diagram showing a stent 100K with a reduced diameter.

The first wire WK1 and the second wire WK2 that are woven into the stent 100K do not intersect at the interlocking portion 2, so they are relatively movable in the longitudinal axis direction A. Therefore, for example, as shown in FIG. 36, when the stent 100K is reduced in diameter and accommodated in the stent delivery system 150, by shifting the interlocking portion 2K1 where the first wires WK1 intersect and the interlocking portion 2K2 where the second wires WK2 intersect in the longitudinal direction A, the outer diameter of the stent 100K becomes smaller. Therefore, the stent 100K can be smoothly contracted and expanded, and can be easily released and recaptured from the stent delivery system 150 .

Next, a method for manufacturing the stent 100K will be explained. In the method for manufacturing a stent of this embodiment, the same jig as in the tenth embodiment is used.

### <First wire weaving step>

FIG. 37 is a developed view of the knitted first wire WK1 developed in the circumferential direction C.

The operator weaves the first wire WK1 along a cylindrical jig with a plurality of projecting pins spirally provided. The operator weaves the first wire WK1 in a zigzag pattern, so as to form the interlocking portion 2 of the second region E2(n-1) and the second region E2(n) on both sides in the longitudinal direction A with the first region E1(n) interposed therebetween.

Specifically, the operator forms the peak-shaped bent portion 3 of the second region E2(n-1) by hooking the first wire WK1 on the protruding pin and bending the first wire WK1. Next, the operator similarly forms the valley-shaped bent portion 4 of the second region E2(n), which is continuous with the recently formed peak-shaped bent portion 3 via the straight-line portion 10. Next, the operator similarly forms the peak-shaped bent portion 3 of the second region E2(n-1), which is connected to the recently formed valley-shaped bent portion 4 via the other straight-line portion 10. Next, the operator similarly forms valley-shaped bent portions 4 in the second region E2(n), which are connected to the most recently formed peak-shaped bent portions 3 via other straight-line portions 10. Thereafter, the operator repeats this to alternately form the peak-shaped bent portions 3 and the valley-shaped bent portions 4, and knits the first wire WK1 along the longitudinal axis direction A.

When weaving the first wires WK1, the operator crosses the first wires WK1 so that the interlocking portion 2 is formed. The peak-shaped bent portion 3 of the first wire WK1 and the valley-shaped bent portion 4 of the first wire WK1 intersect to form an interlocking portion 2K1 where the first wires WK1 intersect each other.

When the weaving of the first wire WK1 is completed, as shown in FIG. 37, the interlocking portion 2 is formed, but the straight-line crossing portion 1 is not formed.

### <Second wire weaving step>

FIG. 38 is a developed view of the knitted first wire WK1 and second wire WK2 developed in the circumferential direction C. The operator weaves the second wire WK2 along a columnar jig provided with a plurality of projecting pins spirally. The operator weaves the second wire WK2 in a zigzag pattern, so as to form the interlocking portion 2 of the second region E2(n-1) and the second region E2(n) on both sides in the longitudinal direction A with the first region E1(n) interposed therebetween.

Specifically, the operator forms the peak-shaped bent portion 3 of the second region E2(n-1) by hooking the second wire WK2 on the protruding pin and bending the second wire WK2. Next, the operator similarly forms the valley-shaped bent portion 4 of the second region E2(n), which is continuous with the recently formed peak-shaped bent portion 3 via the straight-line portion 10. Next, the operator similarly forms the peak-shaped bent portion 3 of the second region E2(n-1), which is connected to the recently formed valley-shaped bent portion 4 via the other straight-line portion 10. Next, the operator similarly forms valley-shaped bent portions 4 in the second region E2(n), which are connected to the most recently formed peak-shaped bent portions 3 via other straight-line portions 10. Thereafter, the operator repeats this to alternately form the peak-shaped bent portions 3 and the valley-shaped bent portions 4, and knits the second wire WK2 along the longitudinal axis direction A.

When weaving the second wires WK2, the operator crosses the second wires WK2 so that the interlocking portion 2 is formed. The peak-shaped bent portion 3 of the second wire WK2 and the valley-shaped bent portion 4 of the second wire WK2 intersect to form an interlocking portion 2K2 where the second wires WK2 intersect each other. Further, when the operator weaves the second wire WK2, the operator crosses the first wire WK1 with the second wire WK2 so that the straight-line crossing portion 1 is formed. The straight-line crossing portion 1 is configured by the straight-line portion 10 of the first wire WK1 and the straight-line portion 10 of the second wire WK2 crossing each other.

### <Marker mounting step>

The operator attaches X-ray visibility markers to predetermined locations on the first wire WK1 and the second wire WK2 as necessary.

### <Washing step>

The operator cleans the woven first wire WK1 and second wire WK2 as necessary.

### <Heat treatment step>

The operator performs heat treatment on the first wire WK1 and the second wire WK2 that are woven, and performs shape memory processing on the first wire WK1 and the second wire WK2. The first wire WK1 and the second wire WK2 are, for example, a superelastic alloy whose main material is NiTi. A superelastic alloy composed mainly of NiTi is not permanently deformed when it is woven, and the woven shape is memorized by applying a heat treatment in a woven state.

### <First bonding step>

The operator joins the end of the first wire WK1 to any part of the first wire WK1 by caulking, laser welding, tight winding, or the like. The operator may remove the first wire WK1 and the second wire WK2 from the jig and then join the ends of the first wire WK1. The operator may join the ends of the first wire WK1 before removing the first wire WK1 and the second wire WK2 from the jig.

### <Second joining step>

The operator joins the end of the second wire WK2 to any part of the second wire WK2 by caulking, laser welding, tight winding, or the like. The operator may remove the first wire WK1 and the second wire WK2 from the jig and then join the ends of the second wire WK2. The operator may join the ends of the second wire WK2 before removing the first wire WK1 and the second wire WK2 from the jig.

In the stent 100K produced by the stent manufacturing method of this embodiment, an odd number of interlocking portions 2 are arranged for each second region E2(n). In the stent 100K illustrated in FIG. 38, 11 interlocking portions 2 are arranged for each second region E2(n). The knitting method of the stent by the stent manufacturing method of the present embodiment is also called "odd number knitting".

According to the stent 100K of the present embodiment, it has a plurality of interlocking portions 2 and has a high shape followability even when it is bent. Since the stent 100K does not need to arrange the straight-line crossing portion 1 at a position adjacent to the interlocking portion 2 in the circumferential direction C, the force (axial force) against bending is reduced. Furthermore, in the stent 100K, the first wire WK1 and the second wire WK2 that are woven are relatively movable in the longitudinal direction A. Therefore, the stent 100K can be smoothly contracted and expanded, and can be easily released and recaptured from the stent delivery system 150.

The eleventh embodiment of the present invention has been described above in detail with reference to the drawings, and the specific configuration is not limited to this embodiment, and design changes and the like are also included within the scope of the present invention. Also, the constituent elements shown in the above-described embodiment and modifications shown below can be combined as appropriate.

### (Modification 11-1)

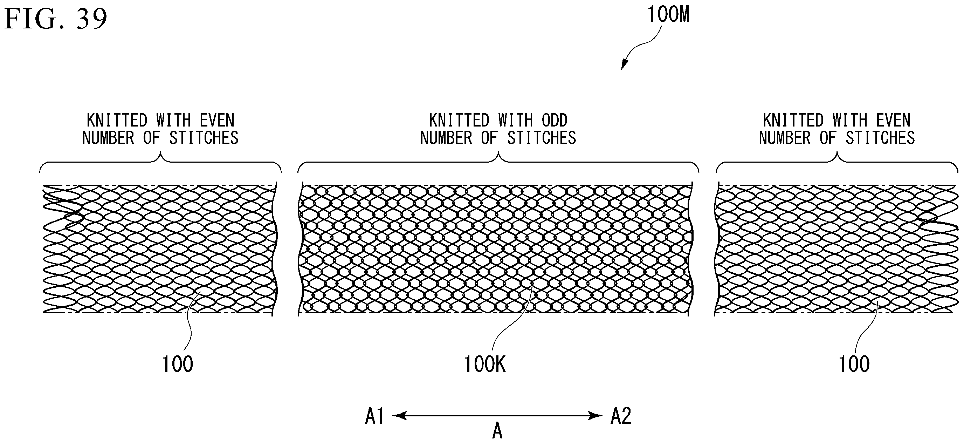

FIG. 39 shows a stent 100M that is a modification of the stent 100K.

The stent 100M is knitted with an odd number of stitches in the central portion in the longitudinal direction A, and is knitted with an even number of stitches at both ends in the longitudinal direction A. The central portion, which is knitted with an odd number of stitches, has a smooth diameter reduction operation similar to the stent 100K, and is easily captured by the stent delivery system 150. As described in the tenth embodiment, both ends knitted by even-numbered knitting have a strong skeleton and can reduce the occurrence of ingrowth and migration. By mixing two types of knitting methods (odd number knitting and even number knitting) with different properties in this way, the stent 100M can have different properties depending on the location.

### (Industrial applicability)

The present invention can be applied to stents formed by weaving wires or the like.

### (Description of the Reference Numerals)

- 300: Endoscope system
- 200: Endoscope
- 150: Stent delivery system
- 110: Outer tubular member
- 120: Inner tubular member
- 130: Chip
- 100, 100B, 100C, 100D, 100E: Stent
- 1: Straight-line crossing portion
- 10: Straight-line portion
- 11: First straight-line portion
- 12: Second straight-line portion
- 1A: Central straight-line crossing portion, First straight-line crossing portion
- 1B: Central straight-line crossing portion, Second straight-line crossing portion
- 1C: Central straight-line crossing portion
- 13: Third straight-line crossing portion
- 14: Fourth straight-line crossing portion
- 15: Fifth straight-line crossing portion
- 2: Interlocking portion (Engaging portion)
- 21: First interlocking portion
- 22: Second interlocking portion
- 23: Third interlocking portion
- 24: Fourth interlocking portion
- 25: Fifth interlocking portion
- 26: Sixth interlocking portion
- 27: Seventh interlocking portion
- 3: Peak-shaped bent portion (Peak)
- 31: First peak
- 32: Second peak
- 33: Third peak
- 34: Fourth peak
- 4: Valley-shaped bent portion (Valley)
- 41: First valley
- 42: Second valley
- 43: Third valley
- 44: Fourth valley

## Claims

1. A stent formed by weaving wires, comprising:
a plurality of straight-line crossing portions, which are formed by crossing at least two straight-line portions of the wires and are arranged adjacent to each other in a circumferential direction of the stent; and
a plurality of interlocking portions configured by intersecting a peak-shaped bent portion, in which the wire is bent in a first direction side which is one side of a longitudinal axis direction of the stent and becomes convex, and a valley-shaped bent portion, in which the wire is bent in a second direction side which is the other side of the longitudinal axis direction and becomes convex, and arranged so as to be adjacent to each other in the circumferential direction of the stent,
wherein the interlocking portions and the straight-line crossing portions are arranged alternately in the longitudinal axis direction.

2. The stent according to claim 1, further comprising:
an upper interlocking portion and a lower interlocking portion, which are the interlocking portions; and
a first straight-line portion that is the straight-line portion continuous with the upper interlocking portion on the first direction side and continuous with the lower interlocking portion on the second direction side,
wherein the first straight-line portion constitutes the straight-line crossing portion with two or more other straight-line portions between the upper interlocking portion and the lower interlocking portion.

3. The stent according to claim 1, wherein
the plurality of straight-line crossing portions include
a first straight-line crossing portion where the first straight-line portion and the second straight-line portion intersect,
a second straight-line crossing portion where the first straight-line portion and the third straight-line portion intersect,
a third straight-line crossing portion where the third straight-line portion and the fourth straight-line portion intersect, and
a fourth straight-line crossing portion where the second straight-line portion and the fifth straight-line portion intersect,
the plurality of interlocking portions include
a first interlocking portion where a first peak, which is the peak-shaped bent portion, and a first valley, which is the valley-shaped bent portion, which are connected to the first direction side of the first straight-line portion intersect,
a second interlocking portion where a second peak, which is the peak-shaped bent portion, and a second valley, which is the valley-shaped bent portion, which are connected to the first direction side of the second straight-line portion and the fourth straight-line portion intersect and
a third interlocking portion where a third peak, which is the peak-shaped bent portion, and a third valley, which is the valley-shaped bent portion, which are connected to the first direction side of the third straight-line portion intersect,
a fourth interlocking portion where a fourth valley, which is the valley-shaped bent portion, and a fourth peak, which is the peak-shaped bent portion, which are connected to the second direction side of the first straight-line portion intersect,
a fifth interlocking portion where a fifth valley, which is the valley-shaped bent portion, and a fifth peak, which is the peak-shaped bent portion, which are connected to the second direction side of the third straight-line portion and the fifth straight-line portion intersect, and
a sixth interlocking portion where a sixth valley, which is the valley-shaped bend, and a sixth peak, which is the peak-shaped bend, which are connected to the second direction side of the second straight-line portion intersect.

4. The stent according to claim 1, wherein
the plurality of straight-line crossing portions include
a first straight-line crossing portion where the first straight-line portion and the second straight-line portion intersect,
a second straight-line crossing portion where the first straight-line portion and the third straight-line portion intersect,
a third straight-line crossing portion where the first straight-line portion and the fourth straight-line portion intersect,
a fourth straight-line crossing portion where the third straight-line portion and the fifth straight-line portion intersect, and
a fifth straight-line crossing portion where the third straight-line portion and the sixth straight-line portion intersect,
the plurality of interlocking portions include
a first interlocking portion where a first peak, which is the peak-shaped bent portion, and a first valley, which is the valley-shaped bent portion, which are connected to the first direction side of the first straight-line portion intersect,
a second interlocking portion where a second peak, which is the peak-shaped bent portion, and a second valley, which is the valley-shaped bent portion, which are connected to the first direction side of the second straight-line portion and the fifth straight-line portion intersect,
a third interlocking portion where a third peak, which is the peak-shaped bent portion, and a third valley, which is the valley-shaped bent portion, which are connected to the first direction side of the third straight-line portion intersect,
a fourth interlocking portion where a fourth valley, which is the valley-shaped bent portion, and a fourth peak, which is the peak-shaped bent portion, which are connected to the second direction side of the first straight-line portion intersect,
a fifth interlocking portion where a fifth valley, which is the valley-shaped bent portion, and a fifth peak, which is the peak-shaped bent portion, which are connected to the second direction side of the fourth straight-line portion and the sixth straight-line portion intersect, and
a sixth interlocking portion where a sixth valley, which is the valley-shaped bend, and a sixth peak, which is the peak-shaped bend, which are connected to the second direction side of the third straight-line portion intersect.

5. The stent according to claim 1, wherein
a first region, in which the plurality of straight-line crossing portions are spirally arranged along the longitudinal axis direction, and a second region, in which the plurality of interlocking portions are spirally arranged along the longitudinal axis direction, are alternately arranged in the longitudinal direction, and
the first regions arranged on both sides in the longitudinal direction across the second region are formed of different wires.

6. The stent according to claim 5, wherein the first regions arranged on both sides of the second region in the longitudinal direction have different numbers of overlapping wires.

7. The stent according to claim 1, wherein
the plurality of straight-line crossing portions have a central straight-line crossing portion where the first straight-line portion of the wire and the second straight-line portion of the wire intersect,
the plurality of interlocking portions include
a first interlocking portion where a first peak, which is the peak-shaped bent portion continuous with the first direction side of the first straight-line portion, and a first valley, which is the valley-shaped bent portion, intersect, and
a second interlocking portion where a second peak, which is the peak-shaped bent portion continuous with the first direction side of the second straight-line portion, and a second valley, which is the valley-shaped bent portion, intersect, and
the first interlocking portion and the second interlocking portion are arranged at different positions in the longitudinal axis direction.

8. The stent according to claim 7, wherein
the wire has a first wire and a second wire different from the first wire,
the first valley and the second peak are formed by the first wire, and
the first peak and the second valley are formed by the second wire.

9. The stent according to claim 7, wherein
the wire has a first wire and a second wire different from the first wire,
the first peak and the first valley are formed by the first wire, and
the second peak and the second valley are formed by the second wire.

10. The stent according to claim 7, wherein
the central straight-line crossing portion is arranged between the first interlocking portion and the second interlocking portion in the circumferential direction of the stent.

11. The stent according to claim 7, wherein
the first interlocking portion is arranged between the second interlocking portion and the central straight-line crossing portion in the longitudinal axis direction.

12. The stent according to claim 7, wherein
the plurality of interlocking portions further include
a third interlocking portion where a third valley, which is the valley-shaped bent portion continuous with the second direction side of the first straight-line portion, and a third peak, which is the peak-shaped bent portion, intersect, and
a fourth interlocking portion where a fourth valley, which is the valley-shaped bent portion continuous with the second direction side of the second straight-line portion, and a fourth peak, which is the peak-shaped bent portion, intersect, and
the third interlocking portion and the fourth interlocking portion are arranged at different positions in the longitudinal axis direction.

13. The stent according to claim 12, wherein the central straight-line crossing portion is arranged between the third interlocking portion and the fourth interlocking portion in the circumferential direction of the stent.

14. The stent according to claim 12, wherein the third interlocking portion is arranged between the central straight-line crossing portion and the fourth interlocking portion in the longitudinal axis direction.

15. The stent according to claim 1, wherein the first regions where the plurality of straight-line crossing portions are arranged and the second regions where the plurality of interlocking portions are arranged are arranged alternately in the longitudinal axis direction.

16. The stent according to claim 15, wherein the first region is spirally arranged along the longitudinal axis.

17. The stent according to claim 15, wherein the second region is spirally arranged along the longitudinal axis.

18. The stent according to claim 12, wherein
in the first interlocking portion, the first peak and the first valley intersect at the first crossing portion and the second crossing portion closer to the central straight-line crossing portion than the first crossing portion,
in the third interlocking portion, the third peak and the third valley intersect at the fourth crossing portion and the third crossing portion closer to the central straight-line crossing portion than the fourth crossing portion,
at the first crossing portion, the first peak passes outside the first valley,
at the second crossing portion, the first peak passes inside the first valley,
at the central straight-line crossing portion, the first straight-line portion passes outside the second straight-line portion,
at the third crossing portion, the third valley passes inside the third peak, and
at the fourth crossing portion, the third valley passes outside the third peak.

19. The stent according to claim 12, wherein
in the first interlocking portion, the first peak and the first valley intersect at the first crossing portion and the second crossing portion closer to the central straight-line crossing portion than the first crossing portion,
in the third interlocking portion, the third peak and the third valley intersect at the fourth crossing portion and the third crossing portion closer to the central straight-line crossing portion than the fourth crossing portion,
at the first crossing portion, the first peak passes inside the first valley,
at the second crossing portion, the first peak passes outside the first valley,
at the central straight-line crossing portion, the first straight-line passes outside the second straight line,
at the third crossing portion, the third valley passes outside the third peak, and
at the fourth crossing portion, the third valley passes inside the third peak.

20. The stent according to claim 12, wherein
in the first interlocking portion, the first peak and the first valley intersect at the first crossing portion and the second crossing portion closer to the central straight-line crossing portion than the first crossing portion,
in the third interlocking portion, the third peak and the third valley intersect at the fourth crossing portion and the third crossing portion closer to the central straight-line crossing portion than the fourth crossing portion,
at the first crossing portion, the first peak passes inside the first valley,
at the second crossing portion, the first peak passes outside the first valley,
at the central straight-line crossing portion, the first straight-line passes outside the second straight line,
at the third crossing portion, the third valley passes inside the third peak, and
at the fourth crossing portion, the third valley passes outside the third peak.

21. The stent according to claim 12, wherein
in the first interlocking portion, the first peak and the first valley intersect at the first crossing portion and the second crossing portion closer to the central straight-line crossing portion than the first crossing portion,
in the third interlocking portion, the third peak and the third valley intersect at the fourth crossing portion and the third crossing portion closer to the central straight-line crossing portion than the fourth crossing portion,
the central straight-line crossing portion includes
a first straight-line crossing portion in which
at the first crossing portion, the first peak passes outside the first valley,
at the second crossing portion, the first peak passes inside the first valley,
at the central straight-line crossing portion, the first straight-line passes outside the second straight line,
at the third crossing portion, the third valley passes inside the third peak, and
at the fourth crossing portion, the third valley passes outside the third peak,
a second straight-line crossing portion in which
at the first crossing portion, the first peak passes inside the first valley,
at the second crossing portion, the first peak passes outside the first valley,
at the central straight-line crossing portion, the first straight-line passes outside the second straight line,
at the third crossing portion, the third valley passes outside the third peak, and
at the fourth crossing portion, the third valley passes inside the third peak.

22. The stent according to claim 21, wherein
the first straight-line crossing portions are arranged continuously in the longitudinal axis direction, and
the second straight-line crossing portion is arranged continuously in the longitudinal axis direction.

23. The stent according to claim 21, wherein the first straight-line crossing portions and the second straight-line crossing portions are arranged alternately in the longitudinal axis direction.

24. The stent according to claim 21, wherein the first straight-line crossing portions are arranged continuously in the circumferential direction.

25. The stent according to claim 21, wherein a predetermined number of the first straight-line crossing portions or the second straight-line crossing portions continuous in the circumferential direction are arranged spirally along the longitudinal axis direction.

26. The stent according to claim 1, wherein the plurality of interlocking portions are arranged adjacent to each other in the circumferential direction of the stent so that the positions in the longitudinal axis direction are different from each other.

27. The stent according to claim 26, wherein the plurality of straight-line crossing portions are arranged adjacent to each other in the circumferential direction of the stent so that the positions in the longitudinal axis direction are different from each other.

28. The stent according to claim 1, wherein
the plurality of interlocking portions include
a fifth interlocking portion,
a sixth interlocking portion adjacent to the fifth interlocking portion in the circumferential direction of the stent, and
a seventh interlocking portion adjacent to the sixth interlocking portion in the circumferential direction of the stent,
wherein positions in the longitudinal axis direction of the fifth interlocking portion and the sixth interlocking portion are the same, and
positions of the sixth interlocking portion and the seventh interlocking portion in the longitudinal axis direction are different.

29. The stent according to claim 28, wherein
the plurality of straight-line crossing portions include
a third straight-line crossing portion,
a fourth straight-line crossing portion adjacent to the third straight-line crossing portion in the circumferential direction of the stent, and
a fifth straight-line crossing portion adjacent to the fourth straight-line crossing portion in the circumferential direction of the stent,
wherein positions in the longitudinal axis direction of the third straight-line crossing portion and the fourth straight-line crossing portion are the same, and
positions in the longitudinal axis direction of the fourth straight-line crossing portion and the fifth straight-line crossing portion are different.

30. A stent delivery system, comprising:
an operation portion;
an outer tubular member configured to extend distally from the operation portion;
an inner tubular member configured to extend distally from the operation portion and located inside the outer tubular member; and
the stent according to any one of claims 1 to 29, which is accommodated between the outer tubular member and the inner tubular member,
wherein the operation portion is configured to place the stent by moving the outer tubular member or the inner tubular member in the longitudinal direction.

31. The stent delivery system according to claim 30, wherein the outer tubular member is configured to be able to be inserted into the channel of the endoscope.

32. A method for manufacturing a stent formed by weaving a first wire and a second wire different from the first wire, the method comprising:
a first wire weaving step of alternately forming a peak-shaped bent portion, which is bent in a first direction that is one side of a longitudinal axis direction of the stent and becomes convex, and a valley-shaped bent portion, which is bent in a second direction that is the other side of the longitudinal axis directions and becomes convex, by weaving the first wire;
a second wire weaving step of weaving a plurality of straight-line crossing portions, where the straight-line portions of the first wire and the straight-line portions of the second wire intersect, and a plurality of interlocking portions, where the peak-shaped bent portion and the valley-shaped bent portion intersect and the first wire and the second wire intersect, by weaving the second wire while intersecting the first wire to alternately form the peak-shaped bent portions and the valley-shaped bent portions; and
a joining step of joining the first wire and the second wire.

33. A method for manufacturing a stent formed by weaving a first wire and a second wire different from the first wire, the method comprising:
a first wire weaving step of weaving a plurality of interlocking portions where the peak-shaped bent portion and the valley-shaped bent portion intersect and the first wires intersect with each other, by weaving the first wire to alternately form a peak-shaped bent portion, which is bent in a first direction that is one side of the longitudinal axis direction of the stent and becomes convex, and a valley-shaped bent portion, which is bent in a second direction that is the other side of the longitudinal axis and becomes convex;
a second wire weaving step of weaving a plurality of straight-line crossing portions, where the straight-line portion of the first wire and the straight-line portion of the second wire intersect, and a plurality of interlocking portions, where the peak-shaped bent portion and the valley-shaped bent portion intersect and the second wires intersect with each other, by weaving the second wire while intersecting the first wire to alternately form the peak-shaped bent portions and the valley-shaped bent portions;
a first joining step of joining the first wires; and
a second joining step of joining the second wires.
